# EUROPEAN PATENT SPECIFICATION

(11) **EP 3 276 009 B1**
(45) Date of publication and mention of the grant of the patent: **09.04.2025**
(21) Application number: 17001302.3
(22) Date of filing: 28.07.2017
(51) Int. Cl.: C12Q 1/68

(54) **MICRORNA EXPRESSION MARKERS FOR CRC DEVELOPMENT**
MICRORNA EXPRESSIONSMARKER FUER DIE ENTWICKLUNG VON DARMKREBS
MICRORNA MARQUEUR D'EXPRESSION DE DEVELOPMENT DU CANCER DU COLON

(30) Priority: 29.07.2016 PL 41814416
(43) Date of publication of application: 31.01.2018
(73) Proprietor: Gdanski Uniwersytet Medyczny, 80-210 Gdansk (PL)
(72) Inventor: Skrzypski, Marcin, 81-721 Sopot (PL); Bobowicz, Maciej, 80-126 Gdansk (PL); Jassem, Jacek, 80-171 Gdansk (PL); Jaskiewicz, Janusz, 80-225 Gdansk (PL)
(74) Representative: JWP Patent & Trademark Attorneys

(56) References cited:
- WO-A1-2012/048236
- WO-A1-2012/115885
- WO-A1-2013/063544
- WO-A2-2013/107460
- CUMMINS JORDAN M ET AL: "The colorectal microRNAome", PROCEEDINGS NATIONAL ACADEMY OF SCIENCES PNAS, NATIONAL ACADEMY OF SCIENCES, US, vol. 103, no. 10, 7 March 2006 (2006-03-07), pages 3687 - 3692, XP002539256, ISSN: 0027-8424, DOI: 10.1073/PNAS.0511155103
- ANONYMOUS: "Abstract 3060: The role of miR-135b in clinical outcome and metastasis in colorectal cancer | Cancer Research", 18 April 2015 (2015-04-18), XP055423155, Retrieved from the Internet <URL:http://cancerres.aacrjournals.org/content/75/15_Supplement/3060> [retrieved on 20171109]
- YANYAN QIU ET AL: "microRNA-497 inhibits invasion and metastasis of colorectal cancer cells by targeting vascular endothelial growth factor-A", CELL PROLIFERATION., vol. 49, no. 1, 1 February 2016 (2016-02-01), GB, pages 69 - 78, XP055423157, ISSN: 0960-7722, DOI: 10.1111/cpr.12237
- BIN HE ET AL: "MicroRNA-155 promotes the proliferation and invasion abilities of colon cancer cells by targeting quaking", MOLECULAR MEDICINE REPORTS, vol. 11, no. 3, 21 November 2014 (2014-11-21), GR, pages 2355 - 2359, XP055423409, ISSN: 1791-2997, DOI: 10.3892/mmr.2014.2994
- ZHAN-JUN LU ET AL: "MicroRNA-185 suppresses growth and invasion of colon cancer cells through inhibition of the hypoxia-inducible factor-2[alpha] pathway in vitro and in vivo", MOLECULAR MEDICINE REPORTS, vol. 10, no. 5, 12 September 2014 (2014-09-12), GR, pages 2401 - 2408, XP055423421, ISSN: 1791-2997, DOI: 10.3892/mmr.2014.2562
- ZHANG JIA-XING ET AL: "Prognostic and predictive value of a microRNA signature in stage II colon cancer: a microRNA expression analysis", THE LANCET ONCOLOGY, vol. 14, no. 13, 1 December 2013 (2013-12-01), AMSTERDAM, NL, pages 1295 - 1306, XP055881862, ISSN: 1470-2045, DOI: 10.1016/S1470-2045(13)70491-1

## Description

### TECHNICAL FIELD

The object of this invention is method of determining distant metastases free survival in colon cancer patients after surgical treatment.

### BACKGROUND ART

WO2012/048236 discloses a technique for the analysis of global miRNA signatures including a larger panel of miRNAs in various groups of well-characterized colorectal cancers (CRCs). However the disclosed diagnosis method is based on obtaining one or more biological samples from the subject and obtaining expression patterns of one or more MicroRNAs (miRNAs) in the biological samples using a microarray, wherein the one or more miRNAs are either upregulated or downregulated in the tissue sample of the subject suspected of having the CRC and comparison with the sample of healthy subject not suffering from CRC. The method does not involve the comparison with prognostic model and determination of the risk score.

WO2012/115885 discloses biomarkers like miRNA which can be assessed for diagnostic, therapy-related or prognostic methods to identify phenotypes, such as a condition or disease, or the stage or progression of a disease. The method does not involve the comparison with prognostic model and determination of the risk score.

WO2013/107460 discloses a method for predicting the efficacy of an anti-angiogenic treatment alone or in combination with chemotherapy an individual suffering from cancer.

WO2012/115885 provides teaching that miRNAs are differentially expressed in CRC and metastases, however the particular microRNA species which refers. to the invention were not disclosed

It is known from "Abstract 3060: The role of miR-135b in clinical outcome and metastasis in colorectal cancer, URL:https://cancerres.aacIjournals.org/content /75/15_Supplement/3060; that the expression level is important with respect to clinical outcome of colorectal cancer and metastasis, but discloses only correlation of miR-135b.

Yanyan Qui et all "microRNA-497 inhibits invasion and metastasis of colorectal cancer cells by targeting vascular endothelial growth factor-A" CELL PROLIFERATION vol.49, no 1, 1 Feb 2016, pages 69-78; ISSN:0960-7722 discloses that marker miR-497 is important in the development of colorectal cancer.

He bin et all "MicroRNA-155 promotes the proliferation and invasion abilities of colon cancer cells by targeting quaking" MOLECULAR MEDICINE REPORTS vol.11, no.3 March 2015, pages 2355-2359, ISSN: 1791-2997 discloses that marker miR-155 is important in the development of colorectal cancer.

LuZhan-Jun et all :MicroRNA-186 suppresses growth and invasion of colon cancer through inhibition of the hypoxia-inducible factor-2-alpha pathway in vitro and in vivo" MOLCEULAR MEDICINE REPORTS vol. 10, no.5 November 2014, pages 2104-2408, ISSN: 1791-2997 discloses that miR-185 was identified as colon cancer growth and metastases suppressing microRNA which functioning as a tumor suppressor.

All mentioned disclosures do not provide teaching for method of detecting of the presence of micrometastases and an increased risk of recurrence of colorectal cancer after a radical surgical treatment.

Zhang Jia-Xing et al: "Prognostic and predictive value of a microRNA signature in stage II colon cancer: a microRNA expression analysis", The Lancet Oncology. Vol. 14, no.13, 1 December 2013 (2013-12-01), pages 1295-1306, XP055881862, Amsterdam NL, ISSN: 1470-2045, DOI: 10.1016/S 1470-2045(13)70491-1 discloses prognostic tool to effectively classify patients with stage II colon cancer into groups at low and high risk of disease recurrence. This tool is based on a six-miRNA signatures being miR-21-5p, miR-20a-5p, miR-103a-3p, miR-106b-5p, miR-43-5p and miR-215.

The prognosis of colorectal cancer in I and II stage is usually good, nevertheless, approx. 20% of them will develop metastases in distant organs (cancer dissemination) within a few years after undergoing a radical operation. At this stage the disease becomes incurable. Applying chemotherapy or immunotherapy after a surgical procedure could prevent a part of recurrences of patients at high risk of cancer dissemination.

Notwithstanding the above, on the basis of the analysis of clinical and microscopic features, it is impossible to distinguish patients with a high risk of cancer dissemination. Due to the knowledge of molecular basics of the appearance and development of tumours, it is currently known that clinical course of a tumour can be conditioned by genes' expression. A test conducted according to the invention consists of a molecular analysis of a fragment of resected tumour in terms of its ability to form metastases.

The method is based on detection in the samples of total RNA obtained from the tumour tissue of short RNA fragments (MicroRNA), and analysing their levels using molecular biology techniques, such as for instance: polymerase chain reaction with the use of reverse transcriptase (RT-PCR), Nano-String, or deep sequencing of transcriptome techniques. The use of the abovementioned method will enable the determination of individual risk of tumour dissemination more precisely, and in consequence, it will be possible to isolate a group of patients with adverse prognosis, among whom an adjuvant therapy could be applied (adjuvant chemotherapy, immunotherapy, or elective treatment).

Adjuvant therapy, which is applied after a surgery, inactivates microscopic metastasis, which are undetectable with the use of currently available diagnostic methods, and thereby it prevents incurable recurrence of the tumour. 180,000 Europeans receive surgical treatment due to colorectal cancer annually, and one in four will experience tumour dissemination. On the one hand, the implementation of a prognostic test indicating patients with an increased risk of recurrence to clinical practice and subjecting them to a post-operative adjuvant therapy could improve the results of treatment in this group, and on the other hand, enable the avoidance of a toxic and unnecessary treatment of patients with good prognosis.

At the current clinical practice, post-operative chemotherapy is not applied in patients who have I-IIA stage of a disease (T2N0-T3N0), due to the fact that 80% of them do not experience tumour recurrence. It is believed that an empirical use of chemotherapy constitutes overtreatment in this group of CC patients. However, such an approach results in the lack of cure in 20% of patients who have an increased risk of recurrence, and who would benefit from post-operative chemotherapy whereby their risk of recurrence would be decreased. Therefore, forgoing adjuvant chemotherapy among these patients is a suboptimal behaviour.

In the case of patients with T4N0 feature, the risk of post-operative cancer dissemination is estimated at approximately 25%. The role of post-operative chemotherapy in this group is controversial, nevertheless this form of treatment is applied due to empirical grounds. This means, however, that 75% of patients having T4N0 stage of disease, who would not have had the recurrence, are subjected to toxic treatment with no clinical benefit.

Currently, available diagnostic method, which enables precise differentiation among people suffering from colorectal cancer in the stage of I-II of a subgroup of patients with an increased risk of metastatic development need to be improved in a way such that the qualification of patients to post-operative chemotherapy on the basis of individually estimated risk of cancer recurrence would be possible. The elaborated assay aims to identify such patients and direct them to proper treatment, which should contribute to the improvement of their results. This approach corresponds to the current paradigm of personalised medicine.

The object of invention is to provide an alternative method based on differential expression of microRNA markers, with which to prognosticate on distant metastasis free survival of colorectal cancer. More particular, the invention provides an assay aimed in evaluation of the risk of recurrence for people suffering from colorectal cancer in the stage I and IIA (T2-T3N0M0), who are subjected to radical surgical treatment, based on the assessment of selected MicroRNA (miRNA) expression.

The study demonstrated expression of 79% of the 750 miRNAs tested, the tested miRNA were included in screening audiometer TaqMan Low Density Arrays A+B3 of LifeTechnologies. The miRNA, of which expression correlated with the tendency to form distant metastasis, was indicated.

On the grounds of the obtained results, a predictive test was prepared - signatures of miRNA expression, which are given by the linear regression formula: logit(RS), by which it is possible to calculate risk index, which is a marker of micrometastases risk and recurrence of colorectal cancer after a post-operative therapy.

As an example, the risk index, which is calculated according to the algorithm weighting the predicting influence of selected miRNA expression for signature 5 of miRNA expression, is highly connected with distant metastasis free survival (DMFS): odds ratio for survival free from tumour recurrence (HR) is 8.4 (95% CI 3.81-18.52); p<0.004, with sensitivity and specificity of detection of metastases 76% and 87%, respectively. Three-year survival rate without metastases for patients with high and low risk of recurrence, which is determined by the aforesaid test was 20% and 81%, respectively. Negative and positive predictive value of the test was 82% and 81%, respectively. In a multifactorial model, to which also tumour's stage was included (pT2 vs. pT3), as well as the stage of its histological differentiation, the risk index turned out to be the only independent predictive factor (HR=8,91 [95% CI 3.69-21.48]).

The predictive value of expression signature was validated positively internally in a *leave-one-out* analysis, in which sensitivity and specificity of prediction of recurrence was estimated on 74% and 78%, respectively. The risk index was also highly connected with overall survival (HR=4.82 [95% CI 2.15-10.77]; p=1.24E-04).

Predictive power of occurring of distant metastases by the predictive test, which was prepared by us (signature of miRNA expression), significantly exceeds the predictive value of conventional clinical-pathological factors of people suffering from colorectal cancer of I and IIA stage. At the same time, there are no useful solutions of this kind on the market.

Test's parameters: Negative and positive predictive value - 82% and 81% respectively (a "training" group), and 74% and 78% respectively for internal validation *leave-one-out.* Odds ratio (HR) of recurrence for patients with High and Low result of risk index was 8.91 (95% CI 3.69-21.48) in this validation.

The purpose of this invention is to make a diagnostic device accessible to patients and doctors, which facilitates the selection of appropriate treatment concerning adjuvant systemic therapy for people suffering from colorectal cancer, who were subjected to a radical surgical treatment (tumour resection) in the I-IIIa stage. On the grounds of the test results, patients with a low risk of recurrence would not be subjected to additional treatment, and patients with a high risk of recurrence would be offered an adjuvant therapy (adjuvant chemotherapy, immunotherapy, or targeted therapies).

The subject of the invention is a method of determining distant metastases free survival in colon cancer patients after surgical treatment consisting of the determination of the quantity of microRNAs comprising isolation of RNA from colorectal cancer tissue and quantification of microRNA, characterised in that 10-microRNAs: hsa-miR-155, hsa-miR-210, hsa-miR-539, hsa-miR-1296, hsa-miR-1303, hsa-miR-151-5p, hsa-miR-497, hsa-miR-552, hsa-miR-572, hsa-miR-939 are quantified by quantitative reverse transcriptase polymerase chain reaction (known as RT-PCR) or hybridisation method, such as Nanostring technology and further the obtained microRNA expression values are input to the microRNA expression values in the recurrence risk prediction model and the risk score RS of recurrence of colorectal cancer is calculated as logit(RS) function including a weighting factor of the contribution of each analysed microRNA according to formula :logit(RS) = 0,95 + 0.86 × hsa-miR-155 + 0.73 × hsa-miR-210 - 0.84 × hsa-miR-539 - 0.47 × hsa-miR-1296 + 0.65 × hsa-miR-1303 - 0,17 × hsa-miR-151-5p - 0,27 × hsa-miR-497 - 0,35 × hsa-miR-552 + 0.55 × hsa-miR-572 - 0,07 × hsa-miR-939.

The second subject of the invention is a method of determining distant metastases free survival in colon cancer patients after surgical treatment consisting of the determination of the quantity of microRNAs comprising isolation of RNA from colorectal cancer tissue and quantification of microRNA, characterised in that 9-microRNAs: hsa-miR-135b, hsa-miR-185, hsa-miR-539, hsa-miR-1296, hsa-miR-572, hsa-miR-939, hsa-miR-155, hsa-miR-497, hsa-miR-1300 are quantified by quantitative reverse transcriptase polymerase chain reaction or hybridisation method, such as Nanostring technology, and further the obtained microRNA expression values are input to the microRNA expression values in the recurrence risk prediction model and the risk score RS of recurrence of colorectal cancer is calculated as logit(RS) function including a weighting factor of the contribution of each analysed microRNA according to formula: logit(RS) = -1,52 - 0.78 × hsa.miR-135b + 1.19 × hsa.miR-185 - 0.57 × hsa.miR-539 - 0.63 × hsa.miR-1296 + 0.61 × hsa.miR-572 + 0,25 × hsa.miR-939 + 0,58 × hsa.miR-155 - 0,35 × hsa.miR-497 + 0.14 × hsa.miR-1300.

The third subject of the invention is a method of determining distant metastases free survival in colon cancer patients after surgical treatment consisting of the determination of the quantity of microRNAs comprising isolation of RNA from colorectal cancer tissue and quantification of microRNA, characterised in that 7-microRNAs: hsa-miR-135b, hsa-miR-185, hsa-miR-539, hsa-miR-1296, hsa-miR-572, hsa-miR-939, hsa-miR-1300 are quantified by quantitative reverse transcriptase polymerase chain reaction or hybridisation method, such as Nanostring technology, and further the obtained microRNA expression values are input to the microRNA expression values in the recurrence risk prediction model and the risk score RS of recurrence of colorectal cancer is calculated as logit(RS) function including a weighting factor of the contribution of each analysed microRNA according to formula: logit(RS) = -2,78 - 0.81 × hsa-miR-135b + 1.45 × hsa-miR-185 - 0.63 × hsa-miR-539 - 0.71 × hsa-miR-1296 + 0.44 × hsa-miR-572 + 0,34 × hsa-miR-939 + 0.13 × hsa-miR-1300;

The forth subject of the invention is a method of determining distant metastases free survival in colon cancer patients after surgical treatment consisting of the determination of the quantity of microRNAs comprising isolation of RNA from colorectal cancer tissue and quantification of microRNA, characterised in that 5-microRNAs: hsa.miR-1296, hsa.miR-135b, hsa.miR-539, hsa.miR-572, hsa.miR-185 are quantified by quantitative reverse transcriptase polymerase chain reaction or hybridisation method, such as Nanostring technology and further the obtained microRNA expression values are input to the microRNA expression values in the recurrence risk prediction model and the risk score RS of recurrence of colorectal cancer is calculated as logit(RS) function including a weighting factor of the contribution of each analysed microRNA according to formula: logit (RS) = -3.61 - 0.72 × hsa-miR-135b + 1.45 × hsa-miR-185 - 0.59 × hsa-miR-539 - 0.61 × hsa-miR-1296 + 0.68 × hsa-miR-572.

The method wherein normalization of expression of each miRNA from the formulas of RS or colon cancer-specific miRNAs, is carried out by the referencing of the prognostic and colon cancer-specific miRNAs to the geometric average of the expression of all or selected miRNAs from the following group of miRNAs: hsa.miR-24, hsa.miR-126, hsa.miR-200c, hsa.miR-26a, hsa.let-7g, hsa.miR-27a, hsa.miR-152, hsa.miR-324-3p, hsa.miR-197, hsa.miR-1274B, hsa.miR-720, hsa.miR-1274A, hsa.miR-1825, hsa.miR-93, hsa.miR-664, hsa.miR-30e-3p, hsa.miR-1285, hsa.miR-335.

The advantages of the method according to the invention includes:
- higher predictive power of cancer recurrence in comparison to other tests,
- higher steadiness of miRNA in comparison with mRNA used in competitive solutions,
- creation of a test with the use of unique, homogeneous clinical material (disease course data).
- The method according to the invention allows for rational selection of a therapy for groups of patients, depending on dissemination risk and also decrease in recurrences frequency after surgical treatment by application of chemotherapy to the group of patiens with high risk of colon cancer recurrency, which leads to increasing of curability by decreasing uncertainty of doctors and patients when making a decision on adjuvant therapy. Also the method of invention allows to select patients with low risk of recurrence of colon cancer and avoid of toxicity and costs connected with unnecessary post-operative chemotherapy for this group of patients.

### FIGURES

### Description of figures and tables:

**Fig. 1** - presents survival time free from distant metastasis (metasis-free survival) depending on a risk index based on the expression signature 5-miRNA, threshold value RS = 0.5 (1A) and chart AUC (area under the curve) for prediction of three-year survival from the surgery without distant metastases according to the risk index based on the expression signature 5-miRNA (1B).
**Fig. 2** **-** presents survival time free from distant metastasis (metasis-free survival) depending on a risk index based on the expression signature 7-miRNA, threshold value RS = 0.53
**Fig. 3** - presents survival time free from distant metastasis (metasis-free survival) depending on a risk index based on the expression signature 9-miRNA, threshold value RS = 0.6

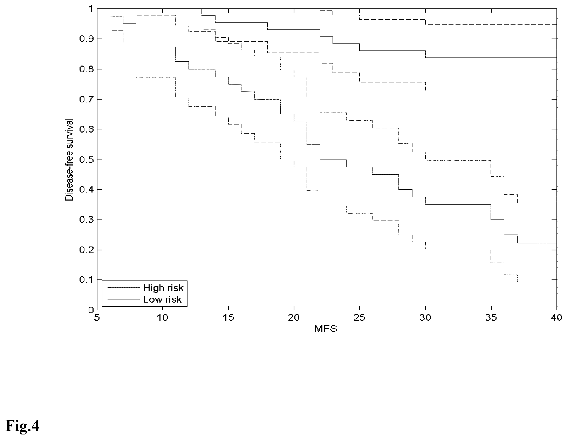

**Fig. 4** - presents survival time free from distant metastasis (metasis-free survival) depending on a risk index based on the expression signature 10-miRNA, threshold value RS = 0.48
**Table 1** [not part of the invention] - presents the characteristics of 85 patients, who had or did not have colorectal cancer recurrence after a surgery. miRNA expression was assessed in the pathomorphological material (sections of tumours), which was the basis for the study of this test. MMR - DNA mismatch repair system - patients with or without impaired mismatch repair system. Clinical-pathological characteristics of the examined group of patients suffering from colorectal cancer in the stage T2-T3N0M0, n=85.
**Table 2** - presents the difference in the level of miRNA expression between the groups of patients with or without colorectal cancer recurrence, where Non-meta = the group, in which there was no tumour recurrence; Meta = the group, in which tumour recurrence occurred; dCt = Ct - geometric mean from Ct for normalising 12miRNA; FC = the multiple of changes; FDR = the statistics correcting false positive results (false discovery rate); HR = odds ratio for survival free from tumour recurrence, CI = confidence interval, (syg) = miRNA conveyed in the predictive signature 5-miRNA
**Table 3** - presents normalised MiRNA used in normalisation of miRNA expression results in colorectal cancer.
**Table 4** - presents differences in miRNA expression between groups of patients with and without colorectal cancer recurrence. FDR - false discovery rate. HR = odds ratio.
**Table 5** - presents miRNA of identification of colorectal cancer and normal colorectal tissue.
**Table 6** - presents miRNA sequences

### EXAMPLES

The invention is illustrated by the following examples of performance, which at the same time do not constitute its limitation.

### EXAMPLE:

The object illustrating the essence of application are the results of miRNA expression in tumour tissue of 85 patients suffering from colorectal cancer in stage T2-3N0, who were subjected to radical surgical treatment and who had metastases after a surgery (tumour recurrence) or who recovered completely (without tumour recurrence). (Table 1). The study was conducted on sections of tumour, which were preserved in formalin and submerged in paraffin wax.

754miRNA expression was analysed with the use of qRT-PCR method. A proprietary method of normalisation of miRNA expression for the purpose of examination of miRNA expression in biological material of people suffering from colorectal cancer was elaborated (Table 3). A proper normalisation is a necessary, integral element of diagnostic testing, based on miRNA expression assessment in order to determine the prognosis of patients who have colorectal cancer. On the grounds of normalised data, miRNA was identified (Table 2 and Table 4), which can contribute to defining prognoses of patients suffering from colorectal cancer. It was stated that expressions miR-939 and miR-1300 are connected with an increased risk of colorectal cancer dissemination in stage T2-T3N0, after the amendment of multiple testing. Moreover, a group of several microRNA was extracted, whose expression identifies tumour tissue (specific expression for tumour tissue), or identifies normal colorectal tissue (specific expression for colon) (Table 5). The inclusion of testing towards these markers constitutes an integral element of diagnostic testing, based on miRNA expression assessment in order to determine the prognosis of patients who have colorectal cancer. It was determined that signature based on expression 5 miRNA, 7 miRNA, 9 miRNA, or 10 miRNA can be a potentially clinically useful predictive marker in this group of patients in relation to cancer dissemination risk.

### METHODS

### Group of patients.

The study has obtained a permission of the Independent Bioethics Committee for Scientific Research at the Medical University of Gdańsk. The analysis involved patients suffering from colorectal cancer (RJG) in stage pT2-3N0, who were subjected to surgical treatment between 2001 and 2011, and who had metastases (tumour recurrence), or who recovered completely (without tumour recurrence). In case of the group of patients without cancer recurrence, the minimal survival time free from disease recurrence was 4 years. Both groups were balanced in terms of clinical and patho-morphological prognostic factors. Patients who had local or nodal recurrence were not included in the study. All patients were subjected to pathological radical hemicolectomy or sigmoidectomy while having lymph node dissection. In order to avoid the effect of hidden metastases to lymph nodes, in all the cases, at least 12 lymph nodes were removed. None of the patients received adjuvant or neoadjuvant chemotherapy or radiotherapy.

### Patho-morphological and molecular analyses

The examined material consisted of sections of removed tumour, which were preserved in formalin and submerged in paraffin wax (FFPE). All tissue blocks were assessed in terms of the confirmation of diagnosis and evaluation of the content percentage of tumour tissue by estimation of a microscopic slide subj ected to haematoxylin and eosin stain (HE) by a patho-morphologist. FFPE block of the highest content of tumour pattern was selected for molecular analyses. In order to further diminish tissue elements' content, which are not included in tumour tissue, elements of normal mucosa of colon, as well as necrotic tissue were excised from FFPE block with the use of macrodissection. After macrodissection, the microscopic slide contained at least 80% of tumour pattern. In order to avoid contamination, each tissue block was cut with the use of a new blade of a microtome.

Four fragments of the thickness of 20 µm, cut from FFPE block were applied to RNA isolation using RecoverAll Kit (Ambion). RNA concentration was assessed in NanoDrop^{®}. Reverse transcription was conducted with the use of 750 ng RNA and TaqManMiRNA RT kit (Applied Biosystems), as well as primers stem-loop pool A and B (Megaplex^{™} PrimerPools, Human Pools Set v3.0, Applied Biosystems), as recommended by the manufacturer. Each pool of primers consists of specific primers towards 377 miRNA, therefore, in order to obtain cDNA for 754 miRNA, the reverse transcription was conducted in two reactions containing in (i) primers of pool A, and in (ii) primers of pool B. With the use of the obtained cDNA, primers specific for miRNA, fluorescent probes TaqMan, and polymerase with the activity of 5' of nuclease, reactions qRT-PCR were carried out in microfluidic cards (TaqMan^{®} ArrayMicrofluidic Cards, Applied Biosystems) in cycler HT 7900 (Applied Biosystems), in conditions recommended by the manufacturer (Applied Biosystems). Raw expression values (Ct) were obtained in application SDS.2.1 (Applied Biosystems).

All FFPE blocks selected for molecular analyses, were also examined in terms of expression of marker proteins for MMR (mismatchrepair) with the use of immune-histochemical methods (IHC) in tissue microarrays (TMAs), which was covered with two tissue fragments, 1.5 mm diameter each. After cutting slides of the thickness of 4 um, TMA was stained in Dakoautostaine on the presence of proteins: MLH1 (clone ES05, Dako, ready to use), MSH2 (G219-1129, Cell Marque, 1:200) and MSH6 (clone EP49, Dako, ready to use). A complete lack of stain reaction (IHC) for MLH1, MSH2, or MSH6 tumour tissue, with preservation of staining in surrounding stroma, was regarded as a determinant of instability of microsatellites (MSI).

The samples of normal colorectal mucosa (NCM) were collected from the area of an operating margin of a microscopic slide, free from tumour in the histopathologic examination.

### Statistical analysis

The primary endpoint was time off from distant metastasis (metastasis-free survival (MFS)). The number of miRNA expression was specified at least in 25%, 50%, 75% and 100% of the samples in both analysed groups (' with recurrence of cancer ' or ' non-recurrence of cancer '). MiRNA with signal amplification (Ct ≥ 40) in fewer than 5% of the samples was included in the prognostic analyses. Missing values of expression (Ct ≥ 40) were imputed in the model "EM-based of the missing data mechanism" [24]. Ct values for miRNA were normalized against the geometric mean for the Ct value of U6 RNA, RNU44, RNU48, and 9 miRNAs, which expression level is the most stable in all tested samples-Norm Finder application (Appendix A, table 1.) [25].

Expression of miRNA was received by means of formula 2 ^-(ΔCt) [26, 27], these values of expression were used to determine times of differences in expression and 95% of confidence ranges. MiRNA expression, specified by ΔCt method, was applied in the subsequent analyses. Lilliefors test was used to verify the hypothesis of normality of the distribution of the signal. Single-factorial analyses were carried out to compare the standardized expression values of miRNA between groups of patients with the use of the nonparametric Mann-Whitney test, and in the analysis of Cox's univariate linear regression, against DMFS values. p values were corrected on multiples of tests with the use of Benjamin-Hochberg's algorithm. All statistical inference was based on threshold of significance alpha = 0.05. Reoccurrence free survival curves (DMFS) were generated using the Kaplan-Meier method. Clinical prognostic value of each miRNA was analysed in the Cox's polyamorous test, to which the following variables were included: the expression of miRNA (as-ΔCt), as well as degree of advancement T and histological differentiation. Negative sign ΔCt allowed for the intuitive interpretation of HR value, for example, in cases where a high expression of miRNA was associated with a high risk of recurrence, the resulting HR value was greater than 1. T feature was expressed in a binary method (pT2 vs. pT3) and degree of histological malignancy as 1, 2 or 3, respectively. Multi-factorial linear regression model was constructed to determine an expression signature, which might characterise the relationship between miRNA expression and the result of treatment (reoccurrence vs. non-recurrence). A risk index was calculated for each patient (recurrence Score (RS)) according to the equation from the model of logistic linear regression. Due to a large number of potential variables, the regression model included miRNA, whose expression was associated with DMFS on the significance level p- value < 0.2 (univariate analyses) [28].

The number of coefficients of the model was specified using the algorithm "forward selection scheme", with the use of a proprietary modulation method based on the Bayesian Information Criterion (BIC), R² and the probability of value ratio p-value that indicates the difference in relation to the model with a smaller number of coefficients. The impact of the individual coefficients was measured with the use of Wald's test. The optimal cut-off threshold RS was found by maximising the positive predictive value (PPV) with a negative predictive value restriction (negative predictive value (NPV)) above 0.8. In addition, regulated parameters of the created model were sought using the LASSO regression model. [29].

A prognostic value of the obtained model was verified by "internal leave-one-out cross-validation" method. A clinical value of the obtained model was also reviewed by means of the Cox's multi-factorial model of the linear regression, to which the following variables were entered: degree of advancement T and histological differentiation. A study involving 85 patients with colorectal cancer, among whom 40 patients experienced reoccurrence of cancer in the form of distant metastasis- 'cases', and 45 patients, who did not experience reoccurrence of cancer -'controls ', has 80% power to detect the difference in survival between groups of "High" and "Low" risk of recurrence of cancer, which is specified according to the size of the test HR = 1.81 with an I error type alpha = 0.05.

In order to find the miRNA, whose expression is specific to colorectal cancer, the data from expression Ct were confounded into the categories of: (I) *no signal of amplification:* Ct ≥ 35, (ii) *expression*: Ct <35. Confounded data were compared between samples of the tumour and normal tissue of the colon by means of the McNamara's test. In order to compare, the expression was normalized as in the section above. The expression miRNA obtained by means of ΔCt method was compared between the groups via T-test for paired data.

### Results

The study concerned 85 patients, including 40 with distant metastasis and 45 without metastasis during at least four years of observation, with an average time of observation of 3.9 and 5.8 years, respectively. The outbreaks of metastasis were located , inter alia, in the lungs, liver, bone and skin. There were no statistically significant differences noted between the groups with and without relapse of the disease with the exception of the disease advancement level (more patients with I stage of advancement were included in the control group; Table 1.). Out of 754 analysed microRNAs, 159 (21%) of the Ct <40 within the limits of 0-5% were considered as a non-expression.

The expression of 465 miRNAs (62%) was reported in at least 25% of the samples, 398 miRNAs (53%) in at least 50% of the samples, 340 miRNAs (45%) in at least 75% of the samples and 99 miRNAs (13%) in all the samples. The expression of 229 miRNAs (30%) was present in more than 95% of the evaluated samples. These miRNAs were evaluated and underwent further analyses. The quality of RNA was typical for FFPE blocks, while Ct values for individual RNA which were used in the standardisation process are shown in Table 3.

### MiRNA expression level and survival time free from distant metastasis

Unifactorial model of Cox's regression has shown a statistically significant relationship between a low expression of miR-1300 and miR-939 and a shorter survival, free from distant metastasis (*distant metastasis free survival,* DMFS) (after correction for multiple comparisons, p=049). Within Cox's multi-factorial prognostic model, which included expression of miRNA, pT and degree of histological malignancy, the low expression of miR-939 (p= 0.011; HR=1,53 [95% CI: 1,10-2.12]) and pT (p=0.05; HR = 2,68 [95% CI: 1.00-7,19]) correlated independently with DMFS. The comparison of medium expressions of miRNA for groups of patients with relapsed disease, and without disease recurrence did not show significant differences after applying the correction on multiple repetitions. In order to identify specific microRNAs potentially involved in the process of metastasis, we searched for the microRNAs whose level of expression was simultaneously correlating with DMFS, (unadjusted Cox test; p <0.05) and differed between the groups of patients with relapsed disease and those without recurrence of disease (the unadjusted U test; p <0,05). Both conditions met the following miRNA miR-1300, miR-939, miR-596, miR-572, miR-210, miR-1303, miR-422a, miR-1260, miR-7, miR-1296, miR-185, miR-26a-1, miR-639, miR-650 and miR-155 (table 2). MiRNA with a prognostic significance or a potentially prognostic significance in colon cancer showed mostly a lower expression in patients with relapsed disease, except miR-1296, which underwent an increased expression. A complete list of miRNA, whose expression correlated with DMFS (Cox's test), and comparison of miRNA expression between groups of patients with relapsed disease and those without disease recurrence (U test) has been shown in Table 4.

### Prognosis Test (1) - REF. expression 5-miRNA

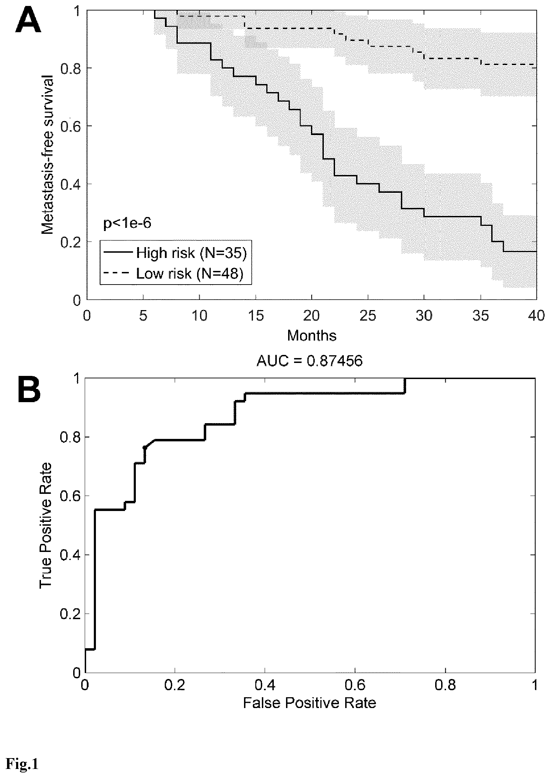

By applying the algorithm, which generates the prognostic model of expression of miRNA, it was possible to create a signature expression of the five microRNAs (5-miRNA 5 signature) undergoing a greater expression (miR-1296, miR-135b and miR539) or less expressive (miR-572 and miR-185) in patients with colon cancer and relapse after the surgical treatment. The contribution of each miRNA along with "weight factors" allowed for the formation the following signature: logit (RS) =-3.61-0.72 × miR-135b 1.45 × miR-185-0.59 x miR-539-0.61 × miR-1296 0.68 x miR-572. The cut off value for the index of risk was: threshold valued RS = 0.5. The risk index of the presented signature was noted to be strongly associated with DMFS (HR = 8.4 [95% CI 3,81-18,52] p< 0.004), upon sensitivity and specificity of the test at the level of 76% and 87% (Fig. 1). Average DMFS in the "high risk" group is estimated at 21 months and has not been reached for a group of "low risk". The three-year DMFS for prognostic groups of "high risk" and "Low-risk" was estimated at 20% and 81% respectively. Negative predictive value (NPV) and positive predictive value (PPV) were estimated at 82% and 81% respectively, and measure of the value of the test different from distribution of chances equalled p=6.28e⁻⁰⁸ (chi-squared test). In Cox's multivariate analysis, covering the odds ratio (risk factor), the degree of clinical malice (combined grades G1 and G2 against grade 3) and advancement level (pT2 pT3 v), the risk ratio was the only variable significantly correlating with DMFS (HR = 8,91 [95% CI 3,69-21,48]. Then, the signature of expression of miRNA underwent cross-validation according to "leave-one-out" method, reaching the sensitivity and specificity of 74% and 78%. The risk coefficient was also correlating with time of survival (HR=4,82 [95% CI 2,15-10,77]; p=1,24E⁻⁰⁴).

### Prognosis Test (2)-expression signature 7-miRNA

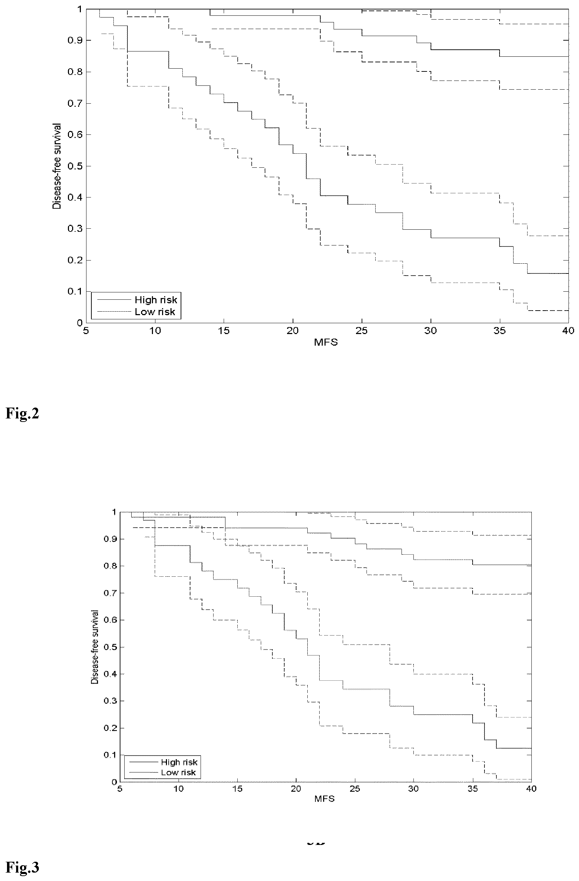

By applying the algorithm, which generates the prognostic model of expression of miRNA, it was possible to create a signature of expression of seven microRNAs (signature 7-miRNA), which either underwent greater expression (miR-1296, miR-135b and miR539) or were less expressive (miR-572 and miR-185, miR-939, miR-1300) in patients with colon cancer and disease relapse after the treatment. The contribution of each miRNA along with "weight factors" allowed the formation of the following signatures: logit(RS) = -2,78 - 0.81 × miR-135b + 1.45 × miR-185 - 0.63 × miR-539 - 0.71 × miR-1296 + 0.44 × miR-572 + 0,34 × miR-939 + 0.13 × miR-1300. The cut off value for the index of risk was: threshold valued RS = 0.53. The index of risk of presented signature was strongly associated with DMFS (HR = 10,83 [95% CI 4,71-24,88] p <0.004), with the sensitivity and specificity of the test at the level of 66% and 78% (Fig. 2). Average DMFS in the "high risk" group amounted to 22 months and has not been reached for a group of "low risk". The three-year DMFS for prognostic groups of "high risk" and "Low-risk" amounted to 20% and 85% respectively. Negative predictive value (NPV) and positive predictive value (PPV) were calculated at 73% and 71% respectively, and the measure of the value of the test different from distribution chances equalled p=1.47e⁻⁰⁷ (chi-squared test). Subsequently, the signature expression of miRNA was subjected to cross-validation according to "leave-one-out" method, reaching the sensitivity and the specificity of 72% and 75% level.

### Prognosis Test (3)-signature of expression of 9-miRNA

By applying the algorithm, which generates the prognostic model of expression of miRNA, it was possible to create a signature of expression of nine microRNAs (9-miRNA signature), which could either be more expressive (miR-1296, miR-135b, miR-539 and miR-497) or less expressive (miR-572, miR-185, miR-939, miR-1300 and miR-155) in patients with colon cancer and a disease relapse after the surgical treatment. The contribution of each miRNA along with "weight factors" allowed the formation of the following signature: logit(RS) = -1,52 - 0.78 × miR-135b + 1.19 × miR-185 - 0.57 × miR-539 - 0.63 × miR-1296 + 0.61 × miR-572 + 0,25 × miR-939 + 0,58 × miR-155 - 0,35 × miR-497 + 0.14 × miR-1300. The cut-off value for the index of risk amounted to: threshold valued RS = 0.6. The index of risk of the presented signature was strongly associated with DMFS (HR = 8,52 [95% CI 4.08-17,79] p<0.004), with the sensitivity and specificity of the test at the level of 57% and 84% (Fig. 3). The average DMFS in the "high risk" group amounted to 21 months and has not been reached by a group of "low risk". The three-year DMFS for prognostic groups of "high risk" and "Low-risk", amounted to 20% and 81% respectively. Negative predictive value (NPV) and positive predictive value (PPV), amounted to 70% and 75% respectively, and the measure of the value of the test different from distribution of chances equalled p=7.33e⁻⁰⁸ (chi-squared test). Then, the signature of expression of miRNA underwent cross-validation according to "leave-one-out" method, reaching the sensitivity and the specificity of 75% and 71% levels.

### Prognosis Test (4)-signature of expression 10-miRNA

By applying the algorithm, which generates the prognostic model of expression of miRNA, it was possible to create a signature of expression of ten microRNAs (10-miRNA signature), which could be either more expressive (miR-1296, miR-135b, miR-552, miR-497, miR-151-5 p) or less expressive (miR-572, miR-185, miR-939, miR-1303 and miR-155, miR-210) in patients with colon cancer and a disease relapse after the surgical treatment. The contribution of each miRNA along with "weight factors", allowed the formation of the following signature: logit(RS) = 0,95 + 0.86 × miR-155 + 0.73 × miR-210 - 0.84 × miR-539 - 0.47 × miR-1296 + 0.65 × miR-1303 - 0,17 × miR-151-5p - 0,27 × miR-497 - 0,35 × miR-552 + 0.55 × miR-572 - 0,07 × miR-939 . The cut-off value for the index of risk was: threshold valued RS = 0.482. The index of risk of the presented signature was strongly associated with DMFS (HR = 8,52 [95% CI 4.08-17,79] p<0.007), with the sensitivity and specificity of the test at the level of 71% and 73% (Fig. 3). The average DMFS in the "high risk" group amounted to 22 months and was not reached by a group of "low risk". The three-year DMFS for prognostic groups of "high risk" and "Low-risk" amounted to 30% and 82% respectively. Negative predictive value (NPV) and positive predictive value (PPV) amounted to 75% and 70% respectively, and the measure of the value of the test different from distribution of chances equalled p=2.13e⁻⁰⁷ (chi-squared test). Then, the signature of expression of miRNA underwent cross-validation according to "leave-one-out" method, reaching the sensitivity and specificity at the levels of 74% and 72%.

### Standardization strategy

For each of the microRNA the value of "stability of expression" was calculated, using the NormFinder algorithm (Andersen et al, 2004). The expression of miRNA in FFPE tissues was noted in a wide range of values of Ct: Ct 17-40. For this reason, it is important to incorporate into the miTNS panel standardization miRNAs, expressed in different expression scopes. In our standardization strategy, 18 miRNAs with stable expression between groups of colon cancer samples from patients with relapse and without relapse were included in the standardization panel. (Table 4). Selected MiRNAs belong to one of three groups of the expression level: standardization miRNAs with a low expression: average value of the Ct >30; standardization miRNAs with the average value of the expression, the average value of Ct >25-30; standardization miRNAs with a high value of expression, average value of Ct ≤ 25. For each group, 3 miRNAs with the lowest value the stability of expression were selected in accordance with the NormFinder algorithm, separately for the pool A (9 standardization miRNAs) and pool B (9 standardization miRNAs).

### Differences in the expression of miRNA between cancer tissue (colorectal cancer) and healthy tissues of the large intestine

MiRNA expression was compared in both, cancer tissue and healthy tissue of the large intestine. By comparing the digitalised values of expression 30 miRNAs were identified with specific expression for colorectal cancer for example, miR-888, miR-523, miR-18b, miR-302a, miR-339-5 p, miR-423-5 p, miR-582-3 p, miR-1243 (p <0.05) and only two miRNAs with specific expression for cancer-free large intestine: miR-299-5 p and miR-1262 (p <0.05) (Table 5)

### LITERATURE

**1.** Torre, L.A., et al., Global cancer statistics, 2012. CA Cancer J Clin, 2015.
**2.** Benson, A.B., et al., American Society of Clinical Oncology recommendations on adjuvant chemotherapy for stage II colon cancer. J Clin Oncol, 2004. 22(16): p. 3408-19.
**3.** Gray, R., et al., Adjuvant chemotherapy versus observation in patients with colorectal cancer: a randomised study. Lancet, 2007. 370(9604): p. 2020-9.
**4.** Marsoni, S. and I.M.P.A.o.C.C.T. Investigators, Efficacy of adjuvant fluorouracil and leucovorin in stage B2 and C colon cancer. International Multicenter Pooled Analysis of Colon Cancer Trials Investigators. Semin Oncol, 2001. 28(1 Suppl 1): p. 14-9.
**5.** Bleeker, W.A., et al., Prognostic significance of K-ras and TP53 mutations in the role of adjuvant chemotherapy on survival in patients with Dukes C colon cancer. Dis Colon Rectum, 2001. 44(3): p. 358-63.
**6.** Chang, M.H., et al., Clinical impact of K-ras mutation in colorectal cancer patients treated with adjuvant FOLFOX. Cancer Chemother Pharmacol, 2011. 68(2): p. 317-23.
**7.** Chen, D., et al., BRAFV600E mutation and its association with clinicopathological features of colorectal cancer: a systematic review and meta-analysis. PLoS One, 2014. 9(3): p. e90607.
**8.** Derbel, O., et al., Impact of KRAS, BRAF and PI3KCA mutations in rectal carcinomas treated with neoadjuvant radiochemotherapy and surgery. BMC Cancer, 2013. 13: p. 200.
**9.** Donada, M., et al., Management of stage II colon cancer - the use of molecular biomarkers for adjuvant therapy decision. BMC Gastroenterol, 2013. 13: p. 36.
**10.** Thibodeau, S.N., G. Bren, and D. Schaid, Microsatellite instability in cancer of the proximal colon. Science, 1993. 260(5109): p. 816-9.
**11.** Merok, M.A., et al., Microsatellite instability has a positive prognostic impact on stage II colorectal cancer after complete resection: results from a large, consecutive Norwegian series. Ann Oncol, 2013. 24(5): p. 1274-82.
**12.** Network, C.G.A., Comprehensive molecular characterization of human colon and rectal cancer. Nature, 2012. 487(7407): p. 330-7.
**13.** Venook, A.P., et al., Biologic determinants of tumor recurrence in stage II colon cancer: validation study of the 12-gene recurrence score in cancer and leukemia group B (CALGB) 9581. J Clin Oncol, 2013. 31(14): p. 1775-81.
**14.** Salazar, R., et al., Gene expression signature to improve prognosis prediction of stage II and III colorectal cancer. J Clin Oncol, 2011. 29(1): p. 17-24.
**15.** Esquela-Kerscher, A. and F.J. Slack, Oncomirs - microRNAs with a role in cancer. Nat Rev Cancer, 2006. 6(4): p. 259-69.
**16.** Fabian, M., N. Sonenberg, and W. Filipowicz, Regulation of mRNA translation and stability by microRNAs. Annu Rev Biochem, 2010. 79: p. 351-79.
**17.** Lewis, B., C. Burge, and D. Bartel, Conserved seed pairing, often flanked by adenosines, indicates that thousands of human genes are microRNA targets. Cell, 2005. 120(1): p. 15-20.
**18.** Szafranska, A.E., et al., Accurate molecular characterization of formalin-fixed, paraffin-embedded tissues by microRNA expression profiling. J Mol Diagn, 2008. 10(5): p. 415-23.
**19.** Zhang, J.X., et al., Prognostic and predictive value of a microRNA signature in stage II colon cancer: a microRNA expression analysis. Lancet Oncol, 2013. 14(13): p. 1295-306.
**20.** Slattery, M.L., et al., An evaluation and replication of miRNAs with disease stage and colorectal cancer-specific mortality. Int J Cancer, 2014.
**21.** Christensen, L.L., et al., MiRNA-362-3p induces cell cycle arrest through targeting of E2F1, USF2 and PTPN1 and is associated with recurrence of colorectal cancer. Int J Cancer, 2013. 133(1): p. 67-78.
**22.** Schee, K., et al., Deep Sequencing the MicroRNA Transcriptome in Colorectal Cancer. PLoS One, 2013. 8(6): p. e66165.
**23.** Schepeler, T., et al., Diagnostic and prognostic microRNAs in stage II colon cancer. Cancer Res, 2008. 68(15): p. 6416-24.
**24.** McCall, M.N., et al., On non-detects in qPCR data. Bioinformatics, 2014. 30(16): p. 2310-6.
**25.** Andersen, C.L., J.L. Jensen, and T.F. Ørntoft, Normalization of real-time quantitative reverse transcription-PCR data: a model-based variance estimation approach to identify genes suited for normalization, applied to bladder and colon cancer data sets. Cancer Res, 2004. 64(15): p. 5245-50.
**26.** Livak, K.J. and T.D. Schmittgen, Analysis of relative gene expression data using real-time quantitative PCR and the 2(-Delta Delta C(T)) Method. Methods, 2001. 25(4): p. 402-8.
**27.** Schmittgen, T.D. and K.J. Livak, Analyzing real-time PCR data by the comparative C(T) method. Nat Protoc, 2008. 3(6): p. 1101-8.
**28.** May, S. and D.W. Hosmer, A cautionary note on the use of the Grønnesby and Borgan goodness-of-fit test for the Cox proportional hazards model. Lifetime Data Anal, 2004. 10(3): p. 283-91.
**29.** Tibshirani, R., The lasso method for variable selection in the Cox model. Stat Med, 1997. 16(4): p. 385-95.
**30.** Goossens-Beumer, I.J., et al., MicroRNA Classifier and Nomogram for Metastasis Prediction in Colon Cancer. Cancer Epidemiol Biomarkers Prev, 2015. 24(1): p. 187-97.
31.Zaykin, D.V., Optimally weighted Z-test is a powerful method for combining probabilities in meta-analysis. J Evol Biol, 2011. 24(8): p. 1836-41.
**32.** Röhr, C., et al., High-throughput miRNA and mRNA sequencing of paired colorectal normal, tumor and metastasis tissues and bioinformatic modeling of miRNA-1 therapeutic applications. PLoS One, 2013. 8(7): p. e67461.
**33.** Marczyk, M., et al., Adaptive filtering of microarray gene expression data based on Gaussian mixture decomposition. BMC Bioinformatics, 2013. 14: p. 101.
**34.** Valeri, N., et al., MicroRNA-135b promotes cancer progression by acting as a downstream effector of oncogenic pathways in colon cancer. Cancer Cell, 2014. 25(4): p. 469-83.
**35.**Lee, J.E., et al., MicroRNA signatures associated with immortalization of EBV-transformed lymphoblastoid cell lines and their clinical traits. Cell Prolif, 2011. 44(1): p. 59-66.
**36.** Muthusamy, S., et al., MicroRNA-539 is up-regulated in failing heart, and suppresses O-GlcNAcase expression. J Biol Chem, 2014. 289(43): p. 29665-76.
**37.** Mi, W., et al., O-GlcNAcylation is a novel regulator of lung and colon cancer malignancy. Biochim Biophys Acta, 2011. 1812(4): p. 514-9.
**38.**Qadir, X.V., et al., miR-185 inhibits hepatocellular carcinoma growth by targeting the DNMT1/PTEN/Akt pathway. Am J Pathol, 2014. 184(8): p. 2355-64.
**39.** Liu, M., et al., miR-185 targets RhoA and Cdc42 expression and inhibits the proliferation potential of human colorectal cells. Cancer Lett, 2011. 301(2): p. 151-60.
**40**. Lu, Y., et al., MicroRNA profiling and prediction of recurrence/relapse-free survival in stage I lung cancer. Carcinogenesis, 2012. 33(5): p. 1046-54.
**41.** Guo, Z., et al., miRNA-939 regulates human inducible nitric oxide synthase posttranscriptional gene expression in human hepatocytes. Proc Natl Acad Sci U S A, 2012. 109(15): p. 5826-31.
**42.** Du, Q., et al., Nitric oxide production upregulates Wnt/β-catenin signaling by inhibiting Dickkopf-1. Cancer Res, 2013. 73(21): p. 6526-37.
**43.** Sarver, A.L., et al., Human colon cancer profiles show differential microRNA expression depending on mismatch repair status and are characteristic of undifferentiated proliferative states. BMC Cancer, 2009. 9: p. 401.
**44.** Tsuchida, A., et al., miR-92 is a key oncogenic component of the miR-17-92 cluster in colon cancer. Cancer Sci, 2011. 102(12): p. 2264-71.
**45.** Neerincx, M., et al., MiR expression profiles of paired primary colorectal cancer and metastases by next-generation sequencing. Oncogenesis, 2015. 4: p. e170.
**46.** Pritchard, C.C., H.H. Cheng, and M. Tewari, MicroRNA profiling: approaches and considerations. Nat Rev Genet, 2012. 13(5): p. 358-69.
**47.** Metzker, M.L., Sequencing technologies - the next generation. Nat Rev Genet, 2010. 11(1): p. 31-46.
**48.** Backes, C., et al., Bias in High-Throughput Analysis of miRNAs and Implications for Biomarker Studies. Anal Chem, 2016. 88(4): p. 2088-95.
**49.** Jorgensen, S., et al., Robust one-day in situ hybridization protocol for detection of microRNAs in paraffin samples using LNA probes. Methods, 2010. 52(4): p. 375-81.
**50.** Nuovo, G.J., In situ detection of precursor and mature microRNAs in paraffin embedded, formalin fixed tissues and cell preparations. Methods, 2008. 44(1): p. 39-46.

**Table 1. Clinical-pathological characteristics of tested group of patients with colorectal cancer in stage T2-T3N0M0, n=85.**

| **miRNA** | **No relapse ΔCt** | | **Relapse ΔCt** | | **Time s diffe rence (KR)** | **KR** | **KR** | **U-test** | | **Cox regression** | | | | |
|---|---|---|---|---|---|---|---|---|---|---|---|---|---|---|
| | **aver age** | **STD** | **aver age** | **STD** | | **low CI** | **hig h CI** | **P-value** | **FDR** | **P-value** | **FD R** | **HR** | **HR 95% CIs** | |
| hsa-miR-1300 | 7.44 | 1.14 | 8.32 | 2.00 | 0.54 | 0.33 | 0.9 0 | 0.035 | 0.634 | <0.00 1 | 0.04 4 | 1.41 1 | 1.17 3 | 1.69 7 |
| hsa-miR-939 | 5.27 | 1.28 | 6.28 | 1.84 | 0.50 | 0.30 | 0.8 1 | 0.014 | 0.549 | <0.00 1 | 0.04 4 | 1.39 4 | 1.15 8 | 1.67 8 |
| hsa-miR-596 | 2.57 | 1.34 | 3.55 | 1.84 | 0.51 | 0.31 | 0.8 3 | 0.010 | 0.549 | 0.001 | 0.06 2 | 1.34 1 | 1.12 2 | 1.60 2 |
| hsa-miR-572 (syg) | 8.52 | 1.39 | 9.65 | 1.91 | 0.45 | 0.27 | 0.7 6 | 0.002 | 0.361 | 0.001 | 0.06 2 | 1.25 2 | 1.09 2 | 1.43 6 |
| hsa-miR-210 | 3.37 | 1.20 | 4.01 | 1.14 | 0.64 | 0.45 | 0.9 2 | 0.012 | 0.549 | 0.012 | 0.30 6 | 1.38 0 | 1.07 3 | 1.77 4 |
| hsa-miR-1303 | 7.80 | 1.30 | 8.73 | 1.93 | 0.52 | 0.32 | 0.8 7 | 0.040 | 0.634 | 0.013 | 0.30 6 | 1.22 2 | 1.04 3 | 1.43 3 |
| hsa-miR-422a | 5.34 | 1.68 | 6.33 | 2.20 | 0.51 | 0.28 | 0.9 2 | 0.032 | 0.634 | 0.014 | 0.30 6 | 1.20 7 | 1.03 9 | 1.40 3 |
| hsa-miR-7 | 6.37 | 1.55 | 7.11 | 1.58 | 0.60 | 0.37 | 0.9 7 | 0.037 | 0.634 | 0.021 | 0.30 6 | 1.21 9 | 1.03 0 | 1.44 2 |
| hsa-miR-1296 (syg) | 9.90 | 1.69 | 9.08 | 1.49 | 1.76 | 1.09 | 2.8 6 | 0.027 | 0.634 | 0.022 | 0.30 6 | 0.77 2 | 0.61 8 | 0.96 4 |

**Table 2. Differences in miRNA expression between groups with cancer reoccurrence and without reoccurrence of colorectal cancer. ΔCt- normalized value of expression, CI-confidence intervals.**

| Variation | Cat. | All patients (N=85) | Group of patients with cancer reoccurren ce (N=40) | Group of patients without cancer reoccurrence (N=45) | p |
|---|---|---|---|---|---|
| Gender | female | 50 | 26 | 24 | 0.275 |
| | male | 35 | 14 | 21 | |
| Degree of advancement | I | 40 | 12 | 28 | 0.003 |
| | IIA | 45 | 28 | 17 | |
| Degree of histological diversification | G1 | 11 | 3 | 8 | 0.187 |
| | G2 | 70 | 34 | 36 | |
| | G3 | 2 | 1 | 1 | |
| Age average (scope) | years | 66 (32-87) | 67 (32-83) | 65 (42-87) | 0.92 |
| Observation time median (range) | years | 3.17 (0.5-11.9) | 1.7 (0.5-3.08) | 4.25 (3.0-11.9) | 8.97e-14 |
| MMR status | MMR incorre ct /all | 13/85 | 6/40 | 7/45 | 0.94 |

**Table 3. Standardization MiRNA with values of expression (raw values Ct) for normalization of expression measurement of miRNA in tumour tissue (primary cancer site of colorectal cancer).**

| **Pot** | **CT ≤25** | | | | **CT 26-30** | | | | **CT >30** | | | |
|---|---|---|---|---|---|---|---|---|---|---|---|---|
| | **miRNA** | **Average value Ct** | **Variance of value Ct** | **Variance coeff. for 2^-ΔCt** | **miRNA** | **Average value Ct t** | **Variance of value Ct** | **Variance coeff. for 2^-ΔCt** | **miRNA** | **Average value Ct** | **Variance of value Ct** | **Variance coeff. for 2^-ΔCt** |
| **A** | miR-24 | 22.9 | 0.933 | 0.546 | miR-26a | 26.7 | 1.305 | 0.603 | miR-152 | 30.3 | 1.444 | 0.626 |
| | miR-126 | 23.4 | 1.162 | 0.558 | let-7g | 27.6 | 1.268 | 0.585 | miR-324-3p | 30.5 | 1.340 | 0.548 |
| | miR-200c | 24.1 | 1.055 | 0.599 | miR-27a | 28.3 | 1.193 | 0.774 | miR-197 | 31.5 | 1.517 | 0.646 |
| | | | | | | | | | | | | |
| **B** | miR-1274B | 18.7 | 1.028 | 0.617 | miR-1825 | 27.4 | 1.195 | 0.627 | miR-30e-3p | 30.1 | 1.117 | 0.616 |
| | miR-720 | 19.7 | 0.752 | 0.556 | miR-93 | 28.6 | 0.801 | 0.501 | miR-1285 | 31.3 | 1.010 | 0.632 |
| | miR-1274A | 22.6 | 1.496 | 0.816 | miR-664 | 28.2 | 0.665 | 0.532 | miR-335# | 32.3 | 2.967 | 0.839 |
| | | | | | | | | | | | | |

**Table 4. Differences in expression of miRNA between groups with reoccurrence and without reoccurrence of colorectal cancer expressed as Times of change in standardized values for group with reoccurrence and without reoccurrence of colorectal cancer. Times of changes=expression in group with recurrence/ expression in group without reoccurrence.**

| **miRNA** | **Times of expression difference** | **low CI** | **high CI** | **U test** | | | **Cox linear regression** | | | | | |
|---|---|---|---|---|---|---|---|---|---|---|---|---|
| | | | | **P-value** | **Q-value** | **FDR** | **P-value** | **Q-value** | **FDR** | **HR** | **HR 95% CI** | |
| hsa-miR-1300 | 0.54 | 0.33 | 0.90 | 3.51E-02 | 7.58E-01 | 6.34E-01 | 2.54E-04 | 4.92E-02 | 4.39E-02 | 1.41 | 1.17 | 1.70 |
| hsa-miR-939 | 0.50 | 0.30 | 0.81 | 1.38E-02 | 6.68E-01 | 5.49E-01 | 4.53E-04 | 4.39E-02 | 4.39E-02 | 1.39 | 1.16 | 1.68 |
| hsa-miR-596 | 0.51 | 0.31 | 0.83 | 1.04E-02 | 1.00E+00 | 5.49E-01 | 1.24E-03 | 8.00E-02 | 6.18E-02 | 1.34 | 1.12 | 1.60 |
| hsa-miR-572 | 0.45 | 0.27 | 0.76 | 1.86E-03 | 3.61E-01 | 3.61E-01 | 1.28E-03 | 6.18E-02 | 6.18E-02 | 1.25 | 1.09 | 1.44 |
| hsa-miR-210 | 0.64 | 0.45 | 0.92 | 1.18E-02 | 7.64E-01 | 5.49E-01 | 1.20E-02 | 4.65E-01 | 3.06E-01 | 1.38 | 1.07 | 1.77 |
| hsa-miR-1303 | 0.52 | 0.32 | 0.87 | 4.02E-02 | 7.09E-01 | 6.34E-01 | 1.32E-02 | 4.27E-01 | 3.06E-01 | 1.22 | 1.04 | 1.43 |
| hsa-miR-422a | 0.51 | 0.28 | 0.92 | 3.21E-02 | 8.90E-01 | 6.34E-01 | 1.39E-02 | 3.86E-01 | 3.06E-01 | 1.21 | 1.04 | 1.40 |
| hsa-miR-1260 | 0.69 | 0.50 | 0.97 | 6.69E-02 | 6.83E-01 | 6.34E-01 | 1.62E-02 | 3.93E-01 | 3.06E-01 | 1.45 | 1.07 | 1.97 |
| hsa-miR-1233 | 0.64 | 0.40 | 1.02 | 3.08E-01 | 7.21E-01 | 6.99E-01 | 1.88E-02 | 4.06E-01 | 3.06E-01 | 1.26 | 1.04 | 1.53 |
| hsa-miR-625 | 0.77 | 0.59 | 0.99 | 8.83E-02 | 6.35E-01 | 6.35E-01 | 2.04E-02 | 3.95E-01 | 3.06E-01 | 1.58 | 1.07 | 2.32 |
| mo-miR-7 | 0.60 | 0.37 | 0.97 | 3.68E-02 | 7.14E-01 | 6.34E-01 | 2.10E-02 | 3.69E-01 | 3.06E-01 | 1.22 | 1.03 | 1.44 |
| hsa-miR-1296 | 1.76 | 1.09 | 2.86 | 2.67E-02 | 8.63E-01 | 6.34E-01 | 2.23E-02 | 3.60E-01 | 3.06E-01 | 0.77 | 0.62 | 0.96 |
| hsa-miR-661 | 0.46 | 0.21 | 1.04 | 5.67E-02 | 6.48E-01 | 6.34E-01 | 2.33E-02 | 3.47E-01 | 3.06E-01 | 1.13 | 1.02 | 1.26 |
| hsa-miR-185 | 0.70 | 0.50 | 0.99 | 8.16E-02 | 6.34E-01 | 6.34E-01 | 2.39E-02 | 3.30E-01 | 3.06E-01 | 1.34 | 1.04 | 1.73 |
| hsa-miR-26a-1 | 0.62 | 0.39 | 0.97 | 7.54E-02 | 6.66E-01 | 6.34E-01 | 2.58E-02 | 3.33E-01 | 3.06E-01 | 1.25 | 1.03 | 1.53 |
| hsa-miR-126 | 0.47 | 0.23 | 0.97 | 8.49E-02 | 6.34E-01 | 6.34E-01 | 2.74E-02 | 3.31E-01 | 3.06E-01 | 1.15 | 1.02 | 1.31 |
| hsa-miR-639 | 0.56 | 0.31 | 1.00 | 1.58E-01 | 7.30E-01 | 6.99E-01 | 2.92E-02 | 3.32E-01 | 3.06E-01 | 1.20 | 1.02 | 1.42 |
| hsa-miR-650 | 0.64 | 0.42 | 0.98 | 4.89E-02 | 6.78E-01 | 6.34E-01 | 2.95E-02 | 3.17E-01 | 3.06E-01 | 1.27 | 1.02 | 1.58 |
| hsa-miR-577 | 0.59 | 0.34 | 1.01 | 3.28E-02 | 7.97E-01 | 6.34E-01 | 3.26E-02 | 3.33E-01 | 3.06E-01 | 1.17 | 1.01 | 1.36 |
| has-miR-155 | 0.62 | 0.43 | 0.89 | 1.41E-02 | 5.49E-01 | 5.49E-01 | 3.29E-02 | 3.18E-01 | 3.06E-01 | 1.29 | 1.02 | 1.64 |
| hsa-miR-636 | 0.46 | 0.21 | 1.03 | 9.53E-02 | 6.61E-01 | 6.61E-01 | 3.40E-02 | 3.14E-01 | 3.06E-01 | 1.16 | 1.01 | 1.32 |
| hsa-miR-571 | 0.61 | 0.36 | 1.05 | 1.63E-01 | 7.38E-01 | 6.99E-01 | 3.48E-02 | 3.06E-01 | 3.06E-01 | 1.20 | 1.01 | 1.42 |
| hsa-miR-25 | 0.73 | 0.49 | 1.07 | 2.91E-01 | 7.25E-01 | 6.99E-01 | 3.78E-02 | 3.18E-01 | 3.18E-01 | 1.33 | 1.02 | 1.75 |
| hsa-miR-191 | 0.82 | 0.66 | 1.01 | 6.29E-02 | 6.78E-01 | 6.34E-01 | 5.35E-02 | 4.31E-01 | 3.93E-01 | 1.54 | 0.99 | 2.38 |
| hsa-miR-203 | 0.68 | 0.47 | 0.98 | 5.55E-02 | 6.74E-01 | 6.34E-01 | 5.41E-02 | 4.19E-01 | 3.93E-01 | 1.27 | 1.00 | 1.63 |
| hsa-miR-497 | 1.83 | 1.09 | 3.08 | 1.30E-01 | 7.91E-01 | 6.99E-01 | 5.70E-02 | 4.24E-01 | 3.93E-01 | 0.83 | 0.69 | 1.01 |
| hsa-miR-19a | 0.59 | 0.34 | 1.04 | 1.05E-01 | 6.78E-01 | 6.78E-01 | 5.77E-02 | 4.14E-01 | 3.93E-01 | 1.17 | 0.99 | 1.37 |
| hsa-miR-151-5P | 1.49 | 1.03 | 2.15 | 6.69E-02 | 6.49E-01 | 6.34E-01 | 5.94E-02 | 4.11E-01 | 3.93E-01 | 0.77 | 0.58 | 1.01 |
| hsa-miR-215 | 0.70 | 0.48 | 1.04 | 4.20E-02 | 6.79E-01 | 6.34E-01 | 6.16E-02 | 4.12E-01 | 3.93E-01 | 1.23 | 0.99 | 1.52 |
| hsa-miR-539 | 1.71 | 1.05 | 2.81 | 2.12E-01 | 7.36E-01 | 6.99E-01 | 6.21E-02 | 4.01E-01 | 3.93E-01 | 0.82 | 0.66 | 1.01 |
| hsa-miR-34a | 0.71 | 0.49 | 1.05 | 1.13E-01 | 7.07E-01 | 6.99E-01 | 6.29E-02 | 3.93E-01 | 3.93E-01 | 1.25 | 0.99 | 1.58 |
| hsa-miR-494 | 0.63 | 0.38 | 1.04 | 7.85E-02 | 6.63E-01 | 6.34E-01 | 6.84E-02 | 4.14E-01 | 4.14E-01 | 1.19 | 0.99 | 1.45 |
| hsa-miR-132 | 0.74 | 0.54 | 1.02 | 4.78E-02 | 7.15E-01 | 6.34E-01 | 7.18E-02 | 4.22E-01 | 4.15E-01 | 1.28 | 0.98 | 1.69 |
| hsa-miR-1225-3P | 0.59 | 0.28 | 1.25 | 2.48E-01 | 7.29E-01 | 6.99E-01 | 7.45E-02 | 4.25E-01 | 4.15E-01 | 1.13 | 0.99 | 1.28 |
| hsa-miR-378 | 0.59 | 0.30 | 1.15 | 1.90E-01 | 7.37E-01 | 6.99E-01 | 7.54E-02 | 4.17E-01 | 4.15E-01 | 1.19 | 0.98 | 1.43 |
| hsa-miR-212 | 0.62 | 0.37 | 1.04 | 5.44E-02 | 7.04E-01 | 6.34E-01 | 7.71E-02 | 4.15E-01 | 4.15E-01 | 1.16 | 0.98 | 1.38 |
| hsa-miR-622 | 0.80 | 0.60 | 1.07 | 2.19E-01 | 7.46E-01 | 6.99E-01 | 8.06E-02 | 4.22E-01 | 4.22E-01 | 1.36 | 0.96 | 1.91 |
| hsa-miR-625 | 0.66 | 0.38 | 1.16 | 3.30E-01 | 7.13E-01 | 6.99E-01 | 8.75E-02 | 4.46E-01 | 4.46E-01 | 1.15 | 0.98 | 1.34 |
| hsa-miR-374b | 0.74 | 0.49 | 1.11 | 1.72E-01 | 7.10E-01 | 6.99E-01 | 9.60E-02 | 4.77E-01 | 4.77E-01 | 1.22 | 0.97 | 1.54 |
| hsa-miR-1290 | 0.75 | 0.50 | 1.13 | 3.30E-01 | 7.05E-01 | 6.99E-01 | 1.06E-01 | 5.15E-01 | 5.15E-01 | 1.21 | 0.96 | 1.54 |
| hsa-miR-744 | 0.79 | 0.58 | 1.08 | 7.10E-02 | 6.57E-01 | 6.34E-01 | 1.23E-01 | 5.81E-01 | 5.58E-01 | 1.26 | 0.94 | 1.69 |
| hsa-miR-320B | 0.74 | 0.41 | 1.32 | 2.29E-01 | 7.19E-01 | 6.99E-01 | 1.30E-01 | 5.98E-01 | 5.58E-01 | 1.13 | 0.96 | 1.33 |
| hsa-miR-140-5p | 0.79 | 0.57 | 1.08 | 2.63E-01 | 7.51E-01 | 6.99E-01 | 1.31E-01 | 5.88E-01 | 5.58E-01 | 1.26 | 0.93 | 1.70 |
| hsa-miR-1183 | 0.74 | 0.45 | 1.21 | 5.68E-01 | 7.99E-01 | 7.82E-01 | 1.33E-01 | 5.86E-01 | 5.58E-01 | 1.17 | 0.95 | 1.43 |
| hsa-miR-20b | 0.68 | 0.40 | 1.16 | 1.55E-01 | 7.54E-01 | 6.99E-01 | 1.36E-01 | 5.86E-01 | 5.58E-01 | 1.13 | 0.96 | 1.32 |
| hsa-miR-425 | 0.79 | 0.58 | 1.09 | 3.78E-01 | 7.34E-01 | 7.19E-01 | 1.38E-01 | 5.82E-01 | 5.58E-01 | 1.25 | 0.93 | 1.68 |
| hsa-miR-106b | 0.77 | 0.50 | 1.17 | 2.09E-01 | 7.65E-01 | 6.99E-01 | 1.40E-01 | 5.78E-01 | 5.58E-01 | 1.17 | 0.95 | 1.43 |
| hsa-miR-18a | 0.67 | 0.41 | 1.10 | 2.44E-01 | 7.29E-01 | 6.99E-01 | 1.42E-01 | 5.73E-01 | 5.58E-01 | 1.14 | 0.96 | 1.36 |
| hsa-miR-638 | 0.71 | 0.41 | 1.21 | 1.58E-01 | 7.48E-01 | 6.99E-01 | 1.44E-01 | 5.69E-01 | 5.58E-01 | 1.13 | 0.96 | 1.33 |
| hsa-miR-374a | 0.73 | 0.43 | 1.22 | 3.00E-01 | 7.36E-01 | 6.99E-01 | 1.47E-01 | 5.71E-01 | 5.58E-01 | 1.15 | 0.95 | 1.38 |
| hsa-miR-29c | 0.56 | 0.23 | 1.34 | 2.55E-01 | 7.40E-01 | 6.99E-01 | 1.49E-01 | 5.64E-01 | 5.58E-01 | 1.08 | 0.97 | 1.21 |
| hsa-miR-410 | 0.75 | 0.50 | 1.12 | 3.00E-01 | 7.27E-01 | 6.99E-01 | 1.50E-01 | 5.58E-01 | 5.58E-01 | 1.18 | 0.94 | 1.49 |
| hsa-miR-552 | 1.78 | 0.93 | 3.39 | 1.69E-01 | 7.46E-01 | 6.99E-01 | 1.59E-01 | 5.80E-01 | 5.72E-01 | 0.89 | 0.76 | 1.05 |
| hsa-miR-140-3p | 0.77 | 0.55 | 1.08 | 1.30E-01 | 7.45E-01 | 6.99E-01 | 1.62E-01 | 5.83E-01 | 5.72E-01 | 1.21 | 0.93 | 1.58 |
| hsa-miR-590-5p | 0.73 | 0.44 | 1.21 | 3.30E-01 | 7.21E-01 | 6.99E-01 | 1.64E-01 | 5.78E-01 | 5.72E-01 | 1.15 | 0.95 | 1.39 |
| hsa-miR-124 | 0.58 | 0.29 | 1.17 | 1.47E-01 | 7.95E-01 | 6.99E-01 | 1.65E-01 | 5.72E-01 | 5.72E-01 | 1.10 | 0.96 | 1.25 |
| hsa-miR-18a | 0.68 | 0.39 | 1.20 | 2.71E-01 | 7.41E-01 | 6.99E-01 | 1.71E-01 | 5.81E-01 | 5.81E-01 | 1.12 | 0.95 | 1.31 |
| hsa-miR-142-3p | 0.73 | 0.47 | 1.15 | 2.12E-01 | 7.49E-01 | 6.99E-01 | 1.75E-01 | 5.84E-01 | 5.81E-01 | 1.15 | 0.94 | 1.41 |
| hsa-miR-500 | 0.75 | 0.47 | 1.19 | 1.78E-01 | 7.19E-01 | 6.99E-01 | 1.77E-01 | 5.81E-01 | 5.81E-01 | 1.14 | 0.94 | 1.37 |
| hsa-miR-328 | 0.64 | 0.32 | 1.28 | 1.05E-01 | 7.01E-01 | 6.78E-01 | 1.82E-01 | 5.88E-01 | 5.88E-01 | 1.09 | 0.96 | 1.25 |
| hsa-miR-1275 | 0.72 | 0.40 | 1.30 | 1.47E-01 | 7.53E-01 | 6.99E-01 | 1.89E-01 | 6.01E-01 | 6.01E-01 | 1.10 | 0.95 | 1.27 |
| hsa-miR-657 | 0.60 | 0.27 | 1.32 | 2.19E-01 | 7.33E-01 | 6.99E-01 | 1.93E-01 | 6.04E-01 | 6.04E-01 | 1.10 | 0.95 | 1.27 |
| hsa-miR-200a | 0.82 | 0.58 | 1.15 | 4.73E-01 | 7.53E-01 | 7.32E-01 | 2.07E-01 | 6.36E-01 | 6.36E-01 | 1.20 | 0.90 | 1.59 |
| hsa-miR-183 | 1.40 | 0.97 | 2.03 | 1.55E-01 | 7.73E-01 | 6.99E-01 | 2.10E-01 | 6.36E-01 | 6.36E-01 | 0.84 | 0.64 | 1.10 |
| hsa-miR-28-3p | 0.85 | 0.65 | 1.11 | 2.83E-01 | 7.13E-01 | 6.99E-01 | 2.21E-01 | 6.60E-01 | 6.60E-01 | 1.23 | 0.88 | 1.70 |
| hsa-miR-135b | 1.42 | 0.85 | 2.37 | 2.02E-01 | 7.56E-01 | 6.99E-01 | 2.28E-01 | 6.70E-01 | 6.69E-01 | 0.89 | 0.73 | 1.08 |
| hsa-miR-888 | 0.54 | 0.19 | 1.57 | 4.79E-01 | 7.50E-01 | 7.32E-01 | 2.34E-01 | 6.77E-01 | 6.69E-01 | 1.05 | 0.97 | 1.14 |
| hsa-miR-21 | 0.80 | 0.54 | 1.17 | 1.84E-01 | 7.28E-01 | 6.99E-01 | 2.36E-01 | 6.72E-01 | 6.69E-01 | 1.14 | 0.92 | 1.42 |
| hsa-miR-486-5p | 0.79 | 0.52 | 1.19 | 1.45E-01 | 8.04E-01 | 6.99E-01 | 2.41E-01 | 6.76E-01 | 6.69E-01 | 1.16 | 0.91 | 1.49 |
| hsa-miR-886-3p | 0.76 | 0.50 | 1.15 | 1.99E-01 | 7.58E-01 | 6.99E-01 | 2.42E-01 | 6.69E-01 | 6.69E-01 | 1.14 | 0.91 | 1.42 |
| hsa-miR-130a | 0.79 | 0.52 | 1.21 | 3.54E-01 | 7.23E-01 | 7.10E-01 | 2.46E-01 | 6.72E-01 | 6.70E-01 | 1.14 | 0.91 | 1.43 |
| hsa-miR-320 | 0.87 | 0.68 | 1.11 | 2.63E-01 | 7.40E-01 | 6.99E-01 | 2.49E-01 | 6.70E-01 | 6.70E-01 | 1.24 | 0.86 | 1.79 |
| hsa-miR-192 | 0.75 | 0.47 | 1.19 | 2.40E-01 | 7.29E-01 | 6.99E-01 | 2.55E-01 | 6.76E-01 | 6.76E-01 | 1.12 | 0.92 | 1.36 |
| hsa-miR-431 | 0.61 | 0.29 | 1.31 | 4.51E-01 | 7.61E-01 | 7.32E-01 | 2.59E-01 | 6.78E-01 | 6.78E-01 | 1.07 | 0.95 | 1.22 |
| hsa-miR-345 | 0.87 | 0.65 | 1.18 | 3.26E-01 | 7.27E-01 | 6.99E-01 | 2.65E-01 | 6.84E-01 | 6.84E-01 | 1.20 | 0.87 | 1.64 |
| hsa-miR-93 | 0.85 | 0.56 | 1.27 | 3.78E-01 | 7.26E-0 1 | 7.19E-01 | 2.74E-01 | 6.98E-01 | 6.98E-01 | 1.13 | 0.91 | 1.40 |
| hsa-miR-362-5p | 0.81 | 0.49 | 1.34 | 2.71E-01 | 7.30E-01 | 6.99E-01 | 2.87E-01 | 7.23E-01 | 7.23E-01 | 1.10 | 0.93 | 1.30 |
| hsa-miR-592 | 1.47 | 0.70 | 3.11 | 2.83E-01 | 7.23E-01 | 6.99E-01 | 2.97E-01 | 7.36E-01 | 7.33E-01 | 0.93 | 0.80 | 1.07 |
| hsa-miR-150 | 0.78 | 0.54 | 1.13 | 8.16E-02 | 6.61E-01 | 6.34E-01 | 3.01E-01 | 7.38E-01 | 7.33E-01 | 1.14 | 0.89 | 1.45 |
| hsa-miR-1253 | 0.77 | 0.42 | 1.42 | 5.08E-01 | 7.47E-01 | 7.47E-01 | 3.09E-01 | 7.48E-01 | 7.33E-01 | 1.08 | 0.93 | 1.27 |
| hsa-miR-34a | 0.84 | 0.58 | 1.22 | 3.35E-01 | 6.99E-01 | 6.99E-01 | 3.10E-01 | 7.41E-01 | 7.33E-01 | 1.14 | 0.89 | 1.46 |
| hsa-miR-186 | 0.87 | 0.65 | 1.15 | 2.37E-01 | 7.29E-01 | 6.99E-01 | 3.11E-01 | 7.35E-01 | 7.33E-01 | 1.18 | 0.85 | 1.64 |
| hsa-miR-487a | 0.67 | 0.34 | 1.31 | 1.47E-01 | 7.74E-01 | 6.99E-01 | 3.17E-01 | 7.39E-01 | 7.33E-01 | 1.07 | 0.94 | 1.22 |
| hsa-miR-99b | 0.85 | 0.60 | 1.21 | 2.26E-01 | 7.19E-01 | 6.99E-01 | 3.22E-01 | 7.42E-01 | 7.33E-01 | 1.16 | 0.87 | 1.55 |
| hsa-miR-195 | 0.82 | 0.56 | 1.20 | 1.69E-01 | 7.29E-01 | 6.99E-01 | 3.25E-01 | 7.40E-01 | 7.33E-01 | 1.12 | 0.90 | 1.40 |
| mo-miR-29c | 0.76 | 0.48 | 1.22 | 6.58E-01 | 8.03E-01 | 7.82E-01 | 3.28E-01 | 7.38E-01 | 7.33E-01 | 1.11 | 0.90 | 1.35 |
| hsa-miR-192 | 0.80 | 0.52 | 1.22 | 2.79E-01 | 7.22E-0 1 | 6.99E-01 | 3.29E-01 | 7.33E-01 | 7.33E-01 | 1.11 | 0.90 | 1.37 |
| hsa-miR-15a | 0.78 | 0.46 | 1.32 | 2.26E-01 | 7.31E-01 | 6.99E-01 | 3.38E-01 | 7.44E-01 | 7.38E-01 | 1.09 | 0.92 | 1.29 |
| hsa-miR-1291 | 1.18 | 0.85 | 1.63 | 3.58E-01 | 7.10E-01 | 7.10E-01 | 3.40E-01 | 7.39E-01 | 7.38E-01 | 0.85 | 0.61 | 1.19 |
| hsa-miR-1201 | 1.27 | 0.86 | 1.88 | 3.04E-01 | 7.28E-01 | 6.99E-01 | 3.45E-01 | 7.43E-01 | 7.38E-01 | 0.89 | 0.71 | 1.13 |
| hsa-miR-134 | 1.27 | 0.84 | 1.92 | 7.80E-01 | 8.33E-01 | 8.16E-01 | 3.51E-01 | 7.48E-01 | 7.38E-01 | 0.89 | 0.70 | 1.13 |
| hsa-miR-125b | 0.80 | 0.51 | 1.24 | 1.30E-01 | 7.67E-01 | 6.99E-01 | 3.52E-01 | 7.42E-01 | 7.38E-01 | 1.10 | 0.90 | 1.34 |
| hsa-miR-452 | 0.85 | 0.57 | 1.26 | 4.40E-01 | 7.63E-01 | 7.32E-01 | 3.59E-01 | 7.48E-01 | 7.38E-01 | 1.12 | 0.88 | 1.41 |
| hsa-miR-484 | 0.72 | 0.37 | 1.42 | 1.69E-01 | 7.13E-01 | 6.99E-01 | 3.66E-01 | 7.55E-01 | 7.38E-01 | 1.07 | 0.93 | 1.22 |
| hsa-miR-133a | 0.72 | 0.37 | 1.41 | 7.87E-01 | 8.22E-01 | 8.16E-01 | 3.69E-01 | 7.53E-01 | 7.38E-01 | 1.07 | 0.92 | 1.24 |
| hsa-miR-223 | 0.80 | 0.54 | 1.19 | 2.79E-01 | 7.32E-01 | 6.99E-01 | 3.70E-01 | 7.46E-01 | 7.38E-01 | 1.11 | 0.88 | 1.41 |
| hsa-miR-23a | 0.74 | 0.37 | 1.51 | 2.12E-01 | 7.63E-01 | 6.99E-01 | 3.71E-01 | 7.40E-01 | 7.38E-01 | 1.06 | 0.93 | 1.22 |
| hsa-miR-146a | 0.85 | 0.63 | 1.15 | 3.04E-01 | 7.20E-0 1 | 6.99E-01 | 3.79E-01 | 7.48E-01 | 7.38E-01 | 1.14 | 0.85 | 1.52 |
| hsa-miR-340 | 0.89 | 0.55 | 1.44 | 8.16E-01 | 8.25E-01 | 8.17E-01 | 3.83E-01 | 7.50E-01 | 7.38E-01 | 1.10 | 0.89 | 1.36 |
| hsa-miR-523 | 0.74 | 0.33 | 1.65 | 5.93E-01 | 8.00E-01 | 7.82E-01 | 3.85E-01 | 7.45E-01 | 7.38E-01 | 1.05 | 0.94 | 1.18 |
| hsa-miR-103 | 0.83 | 0.55 | 1.26 | 3.13E-01 | 7.14E-01 | 6.99E-01 | 3.85E-01 | 7.38E-01 | 7.38E-01 | 1.10 | 0.88 | 1.37 |
| hsa-miR-30a-5p | 1.15 | 0.83 | 1.59 | 5.74E-01 | 7.96E-01 | 7.82E-01 | 3.95E-01 | 7.49E-01 | 7.43E-01 | 0.87 | 0.64 | 1.19 |
| hsa-miR-200a | 0.84 | 0.56 | 1.26 | 3.98E-01 | 7.36E-01 | 7.32E-01 | 3.98E-01 | 7.48E-01 | 7.43E-01 | 1.10 | 0.88 | 1.38 |
| hsa-miR-660 | 0.87 | 0.58 | 1.30 | 6.78E-01 | 7.93E-01 | 7.89E-01 | 4.09E-01 | 7.62E-01 | 7.43E-01 | 1.10 | 0.87 | 1.39 |
| hsa-miR-423-5p | 0.78 | 0.45 | 1.36 | 4.35E-01 | 7.60E-01 | 7.32E-01 | 4.15E-01 | 7.66E-01 | 7.43E-01 | 1.07 | 0.91 | 1.27 |
| hsa-miR-99a | 0.82 | 0.52 | 1.29 | 3.40E-01 | 7.01E-01 | 7.01E-01 | 4.19E-01 | 7.65E-01 | 7.43E-01 | 1.09 | 0.89 | 1.34 |
| hsa-miR-483-3p | 0.88 | 0.42 | 1.85 | 3.58E-01 | 7.17E-01 | 7.10E-01 | 4.21E-01 | 7.61E-01 | 7.43E-01 | 1.06 | 0.92 | 1.21 |
| hsa-miR-532-5p | 0.92 | 0.59 | 1.44 | 9.16E-01 | 8.32E-01 | 8.32E-01 | 4.33E-01 | 7.76E-01 | 7.43E-01 | 1.09 | 0.88 | 1.36 |
| hsa-miR-643 | 0.83 | 0.43 | 1.61 | 3.58E-01 | 7.25E-01 | 7.10E-01 | 4.42E-01 | 7.86E-01 | 7.43E-01 | 1.06 | 0.92 | 1.22 |
| hsa-miR-130b | 0.92 | 0.59 | 1.44 | 3.12E-01 | 7.23E-01 | 6.99E-01 | 4.44E-01 | 7.81E-01 | 7.43E-01 | 1.08 | 0.89 | 1.31 |
| hsa-miR-1247 | 0.79 | 0.34 | 1.82 | 4.29E-01 | 7.58E-01 | 7.32E-01 | 4.45E-01 | 7.77E-01 | 7.43E-01 | 1.05 | 0.92 | 1.20 |
| hsa-miR-331-3p | 0.89 | 0.64 | 1.25 | 3.30E-01 | 7.29E-01 | 6.99E-01 | 4.47E-01 | 7.72E-01 | 7.43E-01 | 1.11 | 0.85 | 1.45 |
| hsa-miR-15b | 0.83 | 0.50 | 1.39 | 2.67E-01 | 7.40E-01 | 6.99E-01 | 4.50E-01 | 7.71E-01 | 7.43E-01 | 1.07 | 0.90 | 1.28 |
| hsa-miR-766 | 0.85 | 0.52 | 1.39 | 3.78E-01 | 7.19E-01 | 7.19E-01 | 4.54E-01 | 7.71E-01 | 7.43E-01 | 1.07 | 0.89 | 1.30 |
| hsa-miR-136 | 1.26 | 0.78 | 2.03 | 6.64E-01 | 7.87E-01 | 7.82E-01 | 4.58E-01 | 7.71E-01 | 7.43E-01 | 0.92 | 0.75 | 1.14 |
| hsa-miR-518f | 0.79 | 0.31 | 2.03 | 5.08E-01 | 7.52E-01 | 7.47E-01 | 4.66E-01 | 7.79E-01 | 7.43E-01 | 1.04 | 0.93 | 1.16 |
| hsa-miR-489 | 0.81 | 0.45 | 1.46 | 4.79E-01 | 7.56E-01 | 7.32E-01 | 4.76E-01 | 7.88E-01 | 7.43E-01 | 1.06 | 0.90 | 1.25 |
| hsa-miR-194 | 0.86 | 0.55 | 1.34 | 4.67E-01 | 7.57E-01 | 7.32E-01 | 4.77E-01 | 7.84E-01 | 7.43E-01 | 1.08 | 0.88 | 1.33 |
| hsa-miR-455-5p | 0.88 | 0.60 | 1.30 | 6.31E-01 | 7.96E-01 | 7.82E-01 | 4.80E-01 | 7.81E-01 | 7.43E-01 | 1.09 | 0.85 | 1.40 |
| hsa-let-7f | 0.89 | 0.53 | 1.52 | 8.87E-01 | 8.29E-01 | 8.22E-01 | 4.80E-01 | 7.75E-01 | 7.43E-01 | 1.07 | 0.89 | 1.28 |
| hsa-miR-28-5p | 0.88 | 0.53 | 1.46 | 7.04E-01 | 8.00E-01 | 7.95E-01 | 4.81E-01 | 7.70E-01 | 7.43E-01 | 1.08 | 0.87 | 1.34 |
| hsa-miR-125a-5p | 0.87 | 0.64 | 1.18 | 6.58E-01 | 7.98E-01 | 7.82E-01 | 4.89E-01 | 7.77E-01 | 7.43E-01 | 1.12 | 0.82 | 1.52 |
| hsa-miR-138 | 0.83 | 0.49 | 1.42 | 7.18E-01 | 8.06E-01 | 8.02E-01 | 4.91E-01 | 7.72E-01 | 7.43E-01 | 1.07 | 0.88 | 1.32 |
| hsa-let-7c | 1.13 | 0.70 | 1.85 | 9.02E-01 | 8.26E-0 1 | 8.22E-01 | 4.94E-01 | 7.71E-01 | 7.43E-01 | 0.93 | 0.77 | 1.14 |
| hsa-miR-584 | 0.76 | 0.35 | 1.66 | 4.45E-01 | 7.59E-01 | 7.32E-01 | 4.94E-01 | 7.66E-01 | 7.43E-01 | 1.04 | 0.92 | 1.18 |
| hsa-miR-491-5p | 0.92 | 0.68 | 1.24 | 7.46E-01 | 8.09E-01 | 8.09E-01 | 5.04E-01 | 7.74E-01 | 7.43E-01 | 1.12 | 0.81 | 1.55 |
| hsa-miR-222 | 0.89 | 0.66 | 1.20 | 4.56E-01 | 7.51E-01 | 7.32E-01 | 5.04E-01 | 7.68E-01 | 7.43E-01 | 1.11 | 0.81 | 1.53 |
| hsa-miR-127-3p | 0.88 | 0.59 | 1.30 | 5.87E-01 | 7.97E-01 | 7.82E-01 | 5.04E-01 | 7.62E-01 | 7.43E-01 | 1.09 | 0.85 | 1.40 |
| hsa-miR-518b | 0.81 | 0.41 | 1.59 | 4.24E-01 | 7.69E-01 | 7.32E-01 | 5.06E-01 | 7.60E-01 | 7.43E-01 | 1.05 | 0.91 | 1.21 |
| hsa-miR-146b-5p | 0.87 | 0.66 | 1.15 | 2.22E-01 | 7.32E-01 | 6.99E-01 | 5.11E-01 | 7.62E-01 | 7.43E-01 | 1.11 | 0.81 | 1.54 |
| hsa-miR-769-5p | 1.10 | 0.79 | 1.53 | 5.43E-01 | 7.70E-01 | 7.70E-01 | 5.13E-01 | 7.59E-01 | 7.43E-01 | 0.90 | 0.67 | 1.22 |
| hsa-miR-30a-3p | 0.90 | 0.67 | 1.23 | 5.19E-01 | 7.53E-01 | 7.53E-01 | 5.14E-01 | 7.54E-01 | 7.43E-01 | 1.11 | 0.82 | 1.49 |
| hsa-miR-143 | 0.86 | 0.57 | 1.30 | 6.06E-01 | 8.11E-01 | 7.82E-01 | 5.15E-01 | 7.50E-01 | 7.43E-01 | 1.08 | 0.86 | 1.35 |
| hsa-miR-17 | 0.89 | 0.60 | 1.31 | 6.25E-01 | 8.04E-01 | 7.82E-01 | 5.16E-01 | 7.46E-01 | 7.43E-01 | 1.08 | 0.85 | 1.38 |
| hsa-miR-411 | 0.85 | 0.53 | 1.36 | 6.06E-01 | 8.06E-01 | 7.82E-01 | 5.24E-01 | 7.52E-01 | 7.43E-01 | 1.06 | 0.88 | 1.29 |
| hsa-miR-19b | 0.85 | 0.52 | 1.37 | 4.24E-01 | 7.77E-01 | 7.32E-01 | 5.27E-01 | 7.51E-01 | 7.43E-01 | 1.07 | 0.87 | 1.30 |
| hsa-let-7b | 0.91 | 0.60 | 1.37 | 3.35E-01 | 7.07E-01 | 6.99E-01 | 5.28E-01 | 7.46E-01 | 7.43E-01 | 1.07 | 0.86 | 1.34 |
| hsa-miR-652 | 0.89 | 0.59 | 1.34 | 3.88E-01 | 7.24E-01 | 7.24E-01 | 5.30E-01 | 7.44E-01 | 7.43E-01 | 1.07 | 0.86 | 1.33 |
| hsa-miR-200b | 0.88 | 0.58 | 1.34 | 6.25E-01 | 7.98E-01 | 7.82E-01 | 5.34E-01 | 7.44E-01 | 7.43E-01 | 1.07 | 0.86 | 1.35 |
| hsa-miR-425 | 1.17 | 0.75 | 1.81 | 8.95E-01 | 8.23E-01 | 8.22E-01 | 5.39E-01 | 7.46E-01 | 7.43E-01 | 0.94 | 0.75 | 1.16 |
| hsa-miR-100 | 0.88 | 0.61 | 1.26 | 6.25E-01 | 8.09E-01 | 7.82E-01 | 5.47E-01 | 7.52E-01 | 7.43E-01 | 1.10 | 0.81 | 1.48 |
| hsa-let-7d | 0.94 | 0.69 | 1.28 | 4.45E-01 | 7.65E-01 | 7.32E-01 | 5.48E-01 | 7.47E-01 | 7.43E-01 | 1.09 | 0.82 | 1.46 |
| hsa-miR-27b | 1.27 | 0.74 | 2.18 | 3.73E-01 | 7.31E-01 | 7.19E-01 | 5.51E-01 | 7.47E-01 | 7.43E-01 | 0.94 | 0.78 | 1.14 |
| hsa-miR-20a | 0.86 | 0.50 | 1.47 | 4.90E-01 | 7.38E-01 | 7.32E-01 | 5.55E-01 | 7.47E-01 | 7.43E-01 | 1.05 | 0.88 | 1.26 |
| hsa-miR-302a | 1.49 | 0.49 | 4.49 | 9.82E-01 | 8.51E-01 | 8.48E-01 | 5.56E-01 | 7.43E-01 | 7.43E-01 | 0.97 | 0.89 | 1.06 |
| hsa-miR-30d | 1.13 | 0.80 | 1.60 | 7.04E-01 | 7.95E-01 | 7.95E-01 | 5.73E-01 | 7.60E-01 | 7.55E-01 | 0.92 | 0.69 | 1.22 |
| hsa-miR-19b-1 | 0.84 | 0.37 | 1.90 | 4.79E-01 | 7.44E-01 | 7.32E-01 | 5.73E-01 | 7.55E-01 | 7.55E-01 | 1.04 | 0.91 | 1.18 |
| hsa-miR-214 | 1.12 | 0.82 | 1.53 | 7.80E-01 | 8.24E-01 | 8.16E-01 | 5.77E-01 | 7.55E-01 | 7.55E-01 | 0.91 | 0.66 | 1.26 |
| hsa-miR-454 | 0.92 | 0.63 | 1.34 | 5.74E-01 | 8.02E-01 | 7.82E-01 | 5.90E-01 | 7.67E-01 | 7.62E-01 | 1.07 | 0.84 | 1.35 |
| hsa-miR-342 | 0.91 | 0.67 | 1.24 | 4.51E-01 | 7.48E-01 | 7.32E-01 | 5.90E-01 | 7.62E-01 | 7.62E-01 | 1.09 | 0.79 | 1.50 |
| hsa-miR-222 | 0.93 | 0.65 | 1.34 | 6.64E-01 | 7.82E-01 | 7.82E-01 | 6.01E-01 | 7.71E-01 | 7.68E-01 | 1.07 | 0.83 | 1.39 |
| hsa-miR-1227 | 0.88 | 0.54 | 1.45 | 8.02E-01 | 8.19E-01 | 8.17E-01 | 6.03E-01 | 7.68E-01 | 7.68E-01 | 1.05 | 0.87 | 1.28 |
| hsa-miR-429 | 0.89 | 0.55 | 1.43 | 7.39E-01 | 8.11E-01 | 8.09E-01 | 6.06E-01 | 7.68E-01 | 7.68E-01 | 1.05 | 0.86 | 1.28 |
| hsa-miR-382 | 0.91 | 0.49 | 1.71 | 4.29E-01 | 7.65E-01 | 7.32E-01 | 6.15E-01 | 7.73E-01 | 7.73E-01 | 1.04 | 0.90 | 1.20 |
| hsa-miR-193b | 1.03 | 0.80 | 1.32 | 6.64E-01 | 7.96E-01 | 7.82E-01 | 6.22E-01 | 7.77E-01 | 7.74E-01 | 0.91 | 0.62 | 1.33 |
| hsa-miR-31 | 0.84 | 0.45 | 1.57 | 4.67E-01 | 7.50E-01 | 7.32E-01 | 6.23E-01 | 7.74E-01 | 7.74E-01 | 1.04 | 0.89 | 1.21 |
| hsa-miR-628-5p | 1.20 | 0.62 | 2.31 | 6.06E-01 | 8.00E-01 | 7.82E-01 | 6.43E-01 | 7.93E-01 | 7.89E-01 | 0.96 | 0.82 | 1.13 |
| hsa-miR-10a | 0.88 | 0.55 | 1.42 | 9.89E-01 | 8.54E-01 | 8.48E-01 | 6.51E-01 | 7.98E-01 | 7.89E-01 | 1.05 | 0.85 | 1.30 |
| hsa-miR-145 | 1.09 | 0.71 | 1.69 | 8.66E-01 | 8.20E-0 1 | 8.20E-0 1 | 6.53E-01 | 7.95E-01 | 7.89E-01 | 0.95 | 0.77 | 1.18 |
| hsa-miR-224 | 1.16 | 0.71 | 1.89 | 3.88E-01 | 7.31E-01 | 7.24E-01 | 6.56E-01 | 7.94E-01 | 7.89E-01 | 0.95 | 0.77 | 1.17 |
| hsa-miR-337-5p | 0.90 | 0.53 | 1.51 | 9.96E-01 | 8.52E-01 | 8.48E-01 | 6.59E-01 | 7.93E-01 | 7.89E-01 | 1.05 | 0.86 | 1.27 |
| hsa-miR-106a | 0.92 | 0.62 | 1.36 | 6.51E-01 | 8.00E-01 | 7.82E-01 | 6.60E-01 | 7.89E-01 | 7.89E-01 | 1.06 | 0.83 | 1.35 |
| hsa-miR-22 | 0.91 | 0.52 | 1.60 | 4.51E-01 | 7.55E-01 | 7.32E-01 | 6.65E-01 | 7.90E-01 | 7.90E-01 | 1.03 | 0.89 | 1.20 |
| hsa-miR-365 | 1.16 | 0.71 | 1.90 | 6.84E-01 | 7.91E-01 | 7.89E-01 | 6.74E-01 | 7.95E-01 | 7.95E-01 | 0.96 | 0.78 | 1.18 |
| hsa-miR-375 | 0.92 | 0.48 | 1.73 | 6.91E-01 | 7.89E-01 | 7.89E-01 | 6.81E-01 | 8.00E-01 | 8.00E-01 | 1.03 | 0.89 | 1.20 |
| hsa-miR-141 | 0.94 | 0.63 | 1.39 | 9.02E-01 | 8.22E-01 | 8.22E-01 | 6.93E-01 | 8.09E-01 | 8.07E-01 | 1.05 | 0.83 | 1.34 |
| hsa-miR-532-3p | 0.97 | 0.66 | 1.43 | 9.89E-01 | 8.50E-01 | 8.48E-01 | 6.96E-01 | 8.07E-01 | 8.07E-01 | 1.05 | 0.82 | 1.34 |
| hsa-miR-10b | 1.13 | 0.69 | 1.84 | 7.59E-01 | 8.15E-01 | 8.15E-01 | 7.05E-01 | 8.12E-01 | 8.07E-01 | 0.96 | 0.78 | 1.18 |
| hsa-miR-26b | 0.93 | 0.56 | 1.54 | 6.84E-01 | 7.96E-01 | 7.89E-01 | 7.08E-01 | 8.12E-01 | 8.07E-01 | 1.04 | 0.86 | 1.25 |
| hsa-miR-605 | 1.15 | 0.64 | 2.06 | 5.43E-01 | 7.76E-01 | 7.70E-01 | 7.10E-01 | 8.09E-01 | 8.07E-01 | 0.97 | 0.82 | 1.14 |
| hsa-miR-181c | 0.95 | 0.55 | 1.65 | 9.74E-01 | 8.60E-01 | 8.48E-01 | 7.12E-01 | 8.07E-01 | 8.07E-01 | 1.03 | 0.87 | 1.23 |
| hsa-miR-214 | 0.94 | 0.70 | 1.25 | 6.12E-01 | 8.03E-01 | 7.82E-01 | 7.31E-01 | 8.23E-01 | 8.11E-01 | 1.06 | 0.76 | 1.47 |
| hsa-miR-95 | 1.11 | 0.68 | 1.81 | 8.16E-01 | 8.17E-01 | 8.17E-01 | 7.34E-01 | 8.21E-01 | 8.11E-01 | 0.97 | 0.79 | 1.18 |
| hsa-miR-34b | 0.97 | 0.62 | 1.51 | 7.32E-01 | 8.08E-01 | 8.08E-01 | 7.55E-01 | 8.41E-01 | 8.11E-01 | 1.03 | 0.84 | 1.28 |
| hsa-miR-1244 | 0.91 | 0.63 | 1.33 | 3.17E-01 | 7.16E-01 | 6.99E-01 | 7.57E-01 | 8.37E-01 | 8.11E-01 | 1.04 | 0.82 | 1.31 |
| hsa-miR-1243 | 0.95 | 0.36 | 2.51 | 2.79E-01 | 7.42E-01 | 6.99E-01 | 7.60E-01 | 8.37E-01 | 8.11E-01 | 1.01 | 0.93 | 1.11 |
| hsa-miR-223 | 1.06 | 0.69 | 1.63 | 5.13E-01 | 7.50E-01 | 7.50E-01 | 7.65E-01 | 8.37E-01 | 8.11E-01 | 0.97 | 0.77 | 1.21 |
| hsa-miR-708 | 0.94 | 0.65 | 1.37 | 6.18E-01 | 8.06E-01 | 7.82E-01 | 7.67E-01 | 8.34E-01 | 8.11E-01 | 1.04 | 0.82 | 1.30 |
| hsa-miR-18b | 0.89 | 0.40 | 1.97 | 4.84E-01 | 7.41E-01 | 7.32E-01 | 7.69E-01 | 8.32E-01 | 8.11E-01 | 1.02 | 0.89 | 1.17 |
| hsa-miR-193a-3p | 1.14 | 0.70 | 1.85 | 8.80E-01 | 8.30E-01 | 8.22E-01 | 7.69E-01 | 8.27E-01 | 8.11E-01 | 0.97 | 0.80 | 1.18 |
| hsa-miR-139-5p | 0.86 | 0.54 | 1.37 | 5.80E-01 | 7.99E-01 | 7.82E-01 | 7.70E-01 | 8.24E-01 | 8.11E-01 | 1.03 | 0.85 | 1.25 |
| hsa-miR-331-5p | 0.95 | 0.34 | 2.69 | 8.59E-01 | 8.30E-01 | 8.20E-0 1 | 7.72E-01 | 8.21E-01 | 8.11E-01 | 1.02 | 0.91 | 1.13 |
| hsa-miR-886-5p | 0.93 | 0.65 | 1.34 | 6.38E-01 | 7.89E-01 | 7.82E-01 | 7.76E-01 | 8.21E-01 | 8.11E-01 | 1.04 | 0.80 | 1.34 |
| hsa-miR-627 | 0.90 | 0.25 | 3.23 | 8.30E-01 | 8.27E-0 1 | 8.20E-0 1 | 7.84E-01 | 8.25E-01 | 8.11E-01 | 1.01 | 0.94 | 1.09 |
| hsa-miR-106b | 1.01 | 0.65 | 1.56 | 4.62E-01 | 7.54E-01 | 7.32E-01 | 7.86E-01 | 8.23E-01 | 8.11E-01 | 1.03 | 0.84 | 1.26 |
| hsa-miR-370 | 0.89 | 0.54 | 1.49 | 5.80E-01 | 7.94E-01 | 7.82E-01 | 7.87E-01 | 8.19E-01 | 8.11E-01 | 1.03 | 0.86 | 1.23 |
| hsa-miR-21 | 0.95 | 0.73 | 1.24 | 4.84E-01 | 7.35E-01 | 7.32E-01 | 7.87E-01 | 8.15E-01 | 8.11E-01 | 1.05 | 0.73 | 1.51 |
| hsa-miR-361-5p | 1.10 | 0.67 | 1.80 | 6.38E-01 | 7.99E-01 | 7.82E-01 | 7.90E-01 | 8.13E-01 | 8.11E-01 | 0.97 | 0.79 | 1.19 |
| hsa-miR-30b | 1.05 | 0.72 | 1.53 | 6.91E-01 | 7.94E-01 | 7.89E-01 | 7.98E-01 | 8.18E-01 | 8.11E-01 | 0.96 | 0.73 | 1.27 |
| hsa-miR-199a-3p | 1.06 | 0.74 | 1.53 | 9.74E-01 | 8.53E-01 | 8.48E-01 | 8.05E-01 | 8.20E-01 | 8.11E-01 | 0.97 | 0.74 | 1.26 |
| hsa-miR-29a | 0.96 | 0.66 | 1.40 | 8.16E-01 | 8.21E-01 | 8.17E-01 | 8.06E-01 | 8.17E-01 | 8.11E-01 | 1.03 | 0.80 | 1.33 |
| hsa-miR-520c-3p | 1.05 | 0.49 | 2.26 | 8.09E-01 | 8.22E-01 | 8.17E-01 | 8.12E-01 | 8.19E-01 | 8.11E-01 | 1.02 | 0.89 | 1.17 |
| hsa-miR-145 | 1.05 | 0.68 | 1.61 | 7.80E-01 | 8.28E-01 | 8.16E-01 | 8.13E-01 | 8.16E-01 | 8.11E-01 | 0.97 | 0.77 | 1.23 |
| hsa-miR-663B | 0.99 | 0.72 | 1.34 | 5.31E-01 | 7.64E-01 | 7.64E-01 | 8.14E-01 | 8.13E-01 | 8.11E-01 | 1.04 | 0.77 | 1.39 |
| hsa-miR-10b | 1.05 | 0.74 | 1.50 | 7.87E-01 | 8.18E-01 | 8.16E-01 | 8.17E-01 | 8.11E-01 | 8.11E-01 | 0.97 | 0.74 | 1.27 |
| hsa-miR-16 | 0.92 | 0.66 | 1.29 | 6.31E-01 | 8.01E-01 | 7.82E-01 | 8.31E-01 | 8.21E-01 | 8.18E-01 | 1.03 | 0.78 | 1.36 |
| hsa-miR-27b | 0.97 | 0.73 | 1.30 | 9.31E-01 | 8.41E-01 | 8.40E-01 | 8.32E-01 | 8.18E-01 | 8.18E-01 | 1.04 | 0.73 | 1.49 |
| hsa-miR-1248 | 1.04 | 0.62 | 1.75 | 9.38E-01 | 8.40E-01 | 8.40E-01 | 8.44E-01 | 8.25E-01 | 8.25E-01 | 0.98 | 0.82 | 1.18 |
| hsa-miR-26b | 0.91 | 0.55 | 1.52 | 4.79E-01 | 7.38E-01 | 7.32E-01 | 8.55E-01 | 8.32E-01 | 8.32E-01 | 1.02 | 0.85 | 1.22 |
| hsa-miR-942 | 1.04 | 0.73 | 1.49 | 8.66E-01 | 8.28E-01 | 8.20E-0 1 | 8.64E-01 | 8.37E-01 | 8.32E-01 | 0.98 | 0.74 | 1.29 |
| hsa-miR-550 | 1.04 | 0.75 | 1.43 | 7.53E-01 | 8.12E-01 | 8.12E-01 | 8.65E-01 | 8.33E-01 | 8.32E-01 | 0.97 | 0.70 | 1.34 |
| hsa-miR-339-5p | 0.96 | 0.56 | 1.65 | 9.53E-01 | 8.45E-01 | 8.45E-01 | 8.68E-01 | 8.32E-01 | 8.32E-01 | 1.02 | 0.84 | 1.23 |
| hsa-miR-92a | 0.96 | 0.65 | 1.41 | 9.38E-01 | 8.43E-01 | 8.40E-01 | 8.73E-01 | 8.33E-01 | 8.33E-01 | 1.02 | 0.79 | 1.31 |
| hsa-miR-483-5p | 1.13 | 0.39 | 3.28 | 8.51E-01 | 8.31E-01 | 8.20E-0 1 | 8.78E-01 | 8.34E-01 | 8.33E-01 | 1.01 | 0.92 | 1.11 |
| hsa-miR-15b | 1.06 | 0.51 | 2.18 | 7.46E-01 | 8.13E-01 | 8.09E-01 | 8.82E-01 | 8.33E-01 | 8.33E-01 | 0.99 | 0.87 | 1.13 |
| hsa-miR-574-3p | 0.96 | 0.74 | 1.24 | 4.90E-01 | 7.32E-01 | 7.32E-01 | 8.89E-01 | 8.35E-01 | 8.35E-01 | 1.03 | 0.71 | 1.48 |
| hsa-miR-339-3p | 0.97 | 0.75 | 1.25 | 7.25E-01 | 8.05E-01 | 8.05E-01 | 8.94E-01 | 8.36E-01 | 8.36E-01 | 1.03 | 0.71 | 1.48 |
| hsa-miR-148b | 0.99 | 0.59 | 1.66 | 8.51E-01 | 8.35E-01 | 8.20E-0 1 | 9.01E-01 | 8.39E-01 | 8.39E-01 | 1.01 | 0.83 | 1.23 |
| hsa-miR-376c | 0.97 | 0.67 | 1.41 | 9.96E-01 | 8.49E-01 | 8.48E-01 | 9.11E-01 | 8.44E-01 | 8.44E-01 | 1.01 | 0.78 | 1.31 |
| hsa-miR-30c | 0.99 | 0.69 | 1.42 | 9.53E-01 | 8.49E-01 | 8.45E-01 | 9.32E-01 | 8.59E-01 | 8.46E-01 | 1.01 | 0.77 | 1.33 |
| hsa-miR-148a | 1.02 | 0.69 | 1.52 | 8.95E-01 | 8.27E-0 1 | 8.22E-01 | 9.32E-01 | 8.55E-01 | 8.46E-01 | 0.99 | 0.76 | 1.29 |
| hsa-miR-29b | 1.09 | 0.64 | 1.85 | 8.37E-01 | 8.29E-01 | 8.20E-0 1 | 9.36E-01 | 8.55E-01 | 8.46E-01 | 0.99 | 0.83 | 1.19 |
| hsa-let-7a | 0.99 | 0.63 | 1.56 | 6.38E-01 | 7.94E-01 | 7.82E-01 | 9.37E-01 | 8.52E-01 | 8.46E-01 | 1.01 | 0.82 | 1.25 |
| hsa-miR-221 | 1.05 | 0.70 | 1.55 | 8.66E-01 | 8.24E-01 | 8.20E-0 1 | 9.46E-01 | 8.56E-01 | 8.46E-01 | 1.01 | 0.80 | 1.27 |
| hsa-let-7e | 1.00 | 0.72 | 1.38 | 8.95E-01 | 8.31E-01 | 8.22E-01 | 9.49E-01 | 8.55E-01 | 8.46E-01 | 1.01 | 0.75 | 1.36 |
| hsa-miR-27a | 0.94 | 0.66 | 1.35 | 4.24E-01 | 7.62E-01 | 7.32E-01 | 9.51E-01 | 8.53E-01 | 8.46E-01 | 1.01 | 0.79 | 1.28 |
| hsa-miR-433 | 1.03 | 0.48 | 2.20 | 6.64E-01 | 7.91E-01 | 7.82E-01 | 9.53E-01 | 8.51E-01 | 8.46E-01 | 1.00 | 0.85 | 1.17 |
| hsa-miR-196b | 1.01 | 0.60 | 1.70 | 9.74E-01 | 8.56E-01 | 8.48E-01 | 9.57E-01 | 8.50E-01 | 8.46E-01 | 1.01 | 0.84 | 1.21 |
| hsa-miR-22 | 0.96 | 0.58 | 1.59 | 9.82E-01 | 8.55E-01 | 8.48E-01 | 9.61E-01 | 8.50E-01 | 8.46E-01 | 1.00 | 0.82 | 1.20 |
| hsa-miR-31 | 1.00 | 0.53 | 1.89 | 8.51E-01 | 8.27E-0 1 | 8.20E-01 | 9.65E-01 | 8.49E-01 | 8.46E-01 | 1.00 | 0.87 | 1.16 |
| hsa-miR-193a-5p | 1.00 | 0.62 | 1.61 | 7.87E-01 | 8.27E-0 1 | 8.16E-01 | 9.65E-01 | 8.46E-01 | 8.46E-01 | 1.00 | 0.82 | 1.23 |
| hsa-miR-182 | 1.07 | 0.64 | 1.77 | 6.58E-01 | 7.93E-01 | 7.82E-01 | 9.72E-01 | 8.48E-01 | 8.46E-01 | 1.00 | 0.82 | 1.21 |
| hsa-miR-151-3p | 1.01 | 0.79 | 1.29 | 8.80E-01 | 8.26E-0 1 | 8.22E-01 | 9.77E-01 | 8.48E-01 | 8.46E-01 | 0.99 | 0.65 | 1.51 |
| hsa-miR-181a | 1.03 | 0.72 | 1.49 | 7.18E-01 | 8.02E-01 | 8.02E-01 | 9.89E-01 | 8.55E-01 | 8.46E-01 | 1.00 | 0.77 | 1.30 |
| hsa-miR-218 | 0.99 | 0.62 | 1.59 | 8.44E-01 | 8.32E-01 | 8.20E-01 | 9.91E-01 | 8.53E-01 | 8.46E-01 | 1.00 | 0.82 | 1.22 |
| hsa-miR-505 | 1.02 | 0.71 | 1.46 | 8.66E-01 | 8.32E-01 | 8.20E-01 | 9.97E-01 | 8.54E-01 | 8.46E-01 | 1.00 | 0.77 | 1.30 |
| hsa-miR-451 | 1.02 | 0.68 | 1.53 | 1.00E+00 | 8.48E-01 | 8.48E-01 | 9.97E-01 | 8.51E-01 | 8.46E-01 | 1.00 | 0.80 | 1.25 |
| hsa-miR-409-3p | 0.99 | 0.74 | 1.34 | 7.94E-01 | 8.16E-01 | 8.16E-01 | 9.99E-01 | 8.49E-01 | 8.46E-01 | 1.00 | 0.72 | 1.38 |
| hsa-miR-376a | 1.02 | 0.68 | 1.53 | 7.94E-01 | 8.21E-01 | 8.16E-01 | 1.00E+00 | 8.46E-01 | 8.46E-01 | 1.00 | 0.80 | 1.25 |

**Table 5. MiRNA with expression specific for colorectal cancer (RJG) and for correct tissue of large intestine (PTJG)**

| | RJG | PTJG |
|---|---|---|
| 1 | hsa-miR-888 | hsa-miR-299-5p |
| 2 | hsa-miR-523 | hsa-miR-1262 |
| 3 | hsa-miR-18b | |
| 4 | hsa-miR-302a | |
| 5 | hsa-miR-339-5p | |
| 6 | hsa-miR-423-5p | |
| 7 | hsa-miR-582-3p | |
| 8 | hsa-miR-181c | |
| 9 | hsa-miR-32 | |
| 10 | hsa-miR-330-3p | |
| 11 | hsa-miR-372 | |
| 12 | hsa-miR-512-3p | |
| 13 | hsa-miR-517a | |
| 14 | hsa-miR-655 | |
| 15 | hsa-miR-182 | |
| 16 | hsa-miR-29b | |
| 17 | hsa-miR-301b | |
| 18 | hsa-miR-382 | |
| 19 | hsa-miR-518f | |
| 20 | hsa-miR-618 | |
| 21 | hsa-miR-577 | |
| 22 | hsa-miR-1243 | |
| 23 | hsa-miR-183 | |
| 24 | hsa-miR-20a | |
| 25 | hsa-miR-31 | |
| 26 | hsa-miR-497 | |
| 27 | hsa-miR-520c-3p | |
| 28 | hsa-miR-591 | |
| 29 | hsa-miR-656 | |
| 30 | hsa-miR-22 | |

**Table 6**

| | Name of microRNA | Sequence of microRNA |
|---|---|---|
| 1 | miR-135b | CACUCUGCUGUGGCCUAUGGCUUUUCAUUCCU AUGUGAUUGCUGUCCCAAACUCAUGUAGGGCU AAAAGCCAUGGGCUACAGUGAGGGGCGAGCUC C |
| 2 | miR-185 | AGGGGGCGAGGGAUUGGAGAGAAAGGCAGUUC CUGAUGGUCCCCUCCCCAGGGGCUGGCUUUCC UCUGGUCCUUCCCUCCCA |
| 3 | miR-539 | AUACUUGAGGAGAAAUUAUCCUUGGUGUGUUC GCUUUAUUUAUGAUGAAUCAUACAAGGACAAU UUCUUUUUGAGUAU |
| 4 | miR. 1296 | ACCUACCUAACUGGGUUAGGGCCCUGGCUCCA UCUCCUUUAGGAAAACCUUCUGUGGGGAGUGG GGCUUCGACCCUAACCCAGGUGGGCUGU |
| 5 | miR-572 | GUCGAGGCCGUGGCCCGGAAGUGGUCGGGCC GCUGCGGGCGGAAGGGCGCCUGUGCUUCGUCC GCUCGGCGGUGGCCCAGCCAGGCCCGCGGGA |
| 6 | miR-939 | UGUGGGCAGGGCCCUGGGGAGCUGAGGCUCUG GGGGUGGCCGGGGCUGACCCUGGGCCUCUGCU CCCCAGUGUCUGACCGCG |
| 7 | miR-1303 | GGCUGGGCAACAUAGCGAGACCUCAACUCUAC AAUUUUUUUUUUUUUUAAAUUUUAGAGACGGGG UCUUGCUCUGUUGCCAGGCUUU |
| 8 | miR-155 | CUGUUAAUGCUAAUCGUGAUAGGGGUUUUUGC CUCCAACUGACUCCUACAUAUUAGCAUUAACA G |
| 9 | miR-497 | CCACCCCGGUCCUGCUCCCGCCCCAGCAGCACA CUGUGGUUUGUACGGCACUGUGGCCACGUCCA AACCACACUGUGGUGUUAGAGCGAGGGUGGGG GAGGCACCGCCGAGG |
| 10 | miR-552 | AACCAUUCAAAUAUACCACAGUUUGUUUAACC UUUUGCCUGUUGGUUGAAGAUGCCUUUCAACA GGUGACUGGUUAGACAAACUGUGGUAUAUACA |
| 11 | miR-572 | GUCGAGGCCGUGGCCCGGAAGUGGUCGGGGCC GCUGCGGGCGGAAGGGCGCCUGUGCUUCGUCC GCUCGGCGGUGGCCCAGCCAGGCCCGCGGGA |
| 12 | miR-1300 | CCACCACUGCUGGCCAUCUGAUCUACAAAUGC AGUGGCAUUGACAAAAGAA CCAUUGAAAAUUUGAGAAGGAGGCUGCUGAGA UGGGA |

## Claims

1. A method of determining distant metastases free survival in colon cancer patients after surgical treatment consisting of determination of the quantity of microRNAs comprising isolation of RNA from colorectal cancer tissue, quantification of microRNA, **characterised in that**
10-microRNAs: hsa-miR-155, hsa-miR-210, hsa-miR-539, hsa-miR-1296, hsa-miR-1303, hsa-miR-151-5p, hsa-miR-497, hsa-miR-552, hsa-miR-572, hsa-miR-939 are quantified by quantitative reverse transcriptase polymerase chain reaction or hybridisation method, such as Nanostring technology, and further the obtained microRNA expression values are input to the microRNA expression values in the recurrence risk prediction model and the risk score RS of recurrence of colorectal cancer is calculated as logit(RS) function including a weighting factor of the contribution of each analysed microRNA according to formula: logit (RS)= 0,95 + 0.86 × hsa-miR-155 + 0.73 × hsa-miR-210 - 0.84 × hsa-miR-539 - 0.47 × hsa-miR-1296 + 0.65 × hsa-miR-1303 - 0,17 × hsa-miR-151-5p - 0,27 × hsa-miR-497 - 0,35 × hsa-miR-552 + 0.55 × hsa-miR-572 - 0,07 × hsa-miR-939.

2. A method of determining distant metastases free survival in colon cancer patients after surgical treatment consisting of determination of the quantity of microRNAs comprising isolation of RNA from colorectal cancer tissue, quantification of microRNA, **characterised in that**
9-microRNAs: hsa-miR-135b, hsa-miR-185, hsa-miR-539, hsa-miR-1296, hsa-miR-572, hsa-miR-939, hsa-miR-155, hsa-miR-497, hsa-miR-1300 are quantified by quantitative reverse transcriptase polymerase chain reaction or hybridisation method, such as Nanostring technology, and further the obtained microRNA expression values are input to the microRNA expression values in the recurrence risk prediction model and the risk score RS of recurrence of colorectal cancer is calculated as logit(RS) function including a weighting factor of the contribution of each analysed microRNA according to the formula: logit(RS) = -1,52 - 0.78 × hsa-miR-135b + 1.19 × hsa-miR-185 - 0.57 × hsa-miR-539 - 0.63 × hsa-miR-1296 + 0.61 × hsa-miR-572 + 0,25 × hsa-miR-939 + 0,58 × hsa-miR-155 - 0,35 × hsa-miR-497 + 0.14 × hsa-miR-1300.

3. A method of determining distant metastases free survival in colon cancer patients after surgical treatment consisting of determination of the quantity of microRNAs comprising isolation of RNA from colorectal cancer tissue, quantification of microRNA, **characterised in that the**
7-microRNAs: hsa-miR-135b, hsa-miR-185, hsa-miR-539, hsa-miR-1296, hsa-miR-572, hsa-miR-939, hsa-miR-1300 are quantified by quantitative reverse transcriptase polymerase chain reaction or hybridisation method, such as Nanostring technology and further the obtained microRNA expression values are input to the microRNA expression values in the recurrence risk prediction model and the risk score RS of recurrence of colorectal cancer is calculated as logit(RS) function including a weighting factor of the contribution of each analysed microRNA according to formula: logit(RS) = -2,78 - 0.81 × hsa-miR-135b + 1.45 × hsa-miR-185 - 0.63 × hsa-miR-539 - 0.71 × hsa-miR-1296 + 0.44 × hsa-miR-572 + 0,34 × hsa-miR-939 + 0.13 × hsa-miR-1300.

4. A method of determining distant metastases free survival in colon cancer patients after surgical treatment consisting of determination of the quantity of microRNAs comprising isolation of RNA from colorectal cancer tissue, quantification of microRNA, **characterised in that the**
5-microRNAs: hsa-miR-1296, hsa-miR-135b, hsa-miR-539, hsa-miR-572, hsa-miR-185 are quantified by quantitative reverse transcriptase polymerase chain reaction or hybridisation method, such as Nanostring technology and further the obtained microRNA expression values are input to the microRNA expression values in the recurrence risk prediction model and the risk score RS of recurrence of colorectal cancer is calculated as logit(RS) function including a weighting factor of the contribution of each analysed microRNA according to formula: logit (RS) = -3.61 - 0.72 × hsa-miR-135b + 1.45 × hsa-miR-185 - 0.59 × hsa-miR-539 - 0.61 × hsa-miR-1296 + 0.68 × hsa-miR-572.

## Patentansprüche

1. Verfahren zur Bestimmung des fernmetastasenfreien Überlebens bei Dickdarmkrebspatienten nach chirurgischer Behandlung, bestehend aus der Bestimmung der Menge an microRNAs, umfassend die Isolierung von RNA aus Dickdarmkrebsgewebe und die Quantifizierung der microRNA, **dadurch gekennzeichnet, dass**
10-microRNAs: hsa-miR-155, hsa-miR-210, hsa-miR-539, hsa-miR-1296, hsa-miR-1303, hsa-miR-151-5p, hsa-miR-497, hsa-miR-552, hsa-miR-572, hsa-miR-939 mittels quantitativer Reverse-Transkriptase-Polymerase-Kettenreaktion oder Hybridisierungsverfahren, wie die Nanostring-Technologie, quantifiziert werden, und ferner werden die erhaltenen microRNA-Expressionswerte in die microRNA-Expressionswerte im Modell zur Vorhersage des Rezidivrisikos eingegeben, und der Risikoscore RS für das Rezidiv von Darmkrebs wird als logit(RS)-Funktion einschließlich eines Gewichtungsfaktors für den Beitrag jeder analysierten microRNA gemäß der Formel berechnet: logit (RS)= 0,95 + 0. 86 × hsa-miR-155 + 0,73 × hsa-miR-210 - 0,84 × hsa-miR-539 - 0,47 × hsa-miR-1296 + 0,65 × hsa-miR-1303 - 0,17 × hsa-miR-151-5p - 0,27 × hsa-miR-497 - 0,35 × hsa-miR-552 + 0,55 × hsa-miR-572 - 0,07 × hsa-miR-939.

2. Verfahren zur Bestimmung des fernmetastasenfreien Überlebens bei Dickdarmkrebspatienten nach chirurgischer Behandlung, bestehend aus der Bestimmung der Menge an microRNAs, umfassend die Isolierung von RNA aus Dickdarmkrebsgewebe und die Quantifizierung der microRNA, **dadurch gekennzeichnet, dass**
9-microRNAs:: hsa-miR-135b, hsa-miR-185, hsa-miR-539, hsa-miR-1296, hsa-miR-572, hsa-miR-939, hsa-miR-155, hsa-miR-497, hsa-miR-1300 mittels quantitativer Reverse-Transkriptase-Polymerase-Kettenreaktion oder Hybridisierungsverfahren, wie die Nanostring-Technologie, quantifiziert werden, und ferner werden die erhaltenen microRNA-Expressionswerte in die microRNA-Expressionswerte im Modell zur Vorhersage des Rezidivrisikos eingegeben, und der Risikoscore RS für das Rezidiv von Darmkrebs wird als logit(RS)-Funktion einschließlich eines Gewichtungsfaktors für den Beitrag jeder analysierten microRNA gemäß der Formel berechnet: logit(RS) = - 1,52 - 0.78 × hsa-miR-135b + 1,19 × hsa-miR-185 - 0,57 × hsa-miR-539 - 0,63 × hsa-miR-1296 + 0,61 × hsa-miR-572 + 0,25 × hsa-miR-939 + 0,58 × hsa-miR-155 - 0,35 × hsa-miR-497 + 0,14 × hsa-miR-1300.

3. Verfahren zur Bestimmung des fernmetastasenfreien Überlebens bei Dickdarmkrebspatienten nach chirurgischer Behandlung, bestehend aus der Bestimmung der Menge an microRNAs, umfassend die Isolierung von RNA aus Dickdarmkrebsgewebe und die Quantifizierung der microRNA, **dadurch gekennzeichnet, dass**
7-microRNAs: hsa-miR-135b, hsa-miR-185, hsa-miR-539, hsa-miR-1296, hsa-miR-572, hsa-miR-939, hsa-miR-1300 mittels quantitativer Reverse-Transkriptase-Polymerase-Kettenreaktion oder Hybridisierungsverfahren, wie die Nanostring-Technologie, quantifiziert werden, und ferner werden die erhaltenen microRNA-Expressionswerte in die microRNA-Expressionswerte im Modell zur Vorhersage des Rezidivrisikos eingegeben, und der Risikoscore RS für das Rezidiv von Darmkrebs wird als logit(RS)-Funktion einschließlich eines Gewichtungsfaktors für den Beitrag jeder analysierten microRNA gemäß der Formel berechnet: logit(RS) = -2,78 - 0. 81 × hsa-miR-135b + 1,45 × hsa-miR-185 - 0,63 × hsa-miR-539 - 0,71 × hsa-miR-1296 + 0,44 × hsa-miR-572 + 0,34 × hsa-miR-939 + 0,13 × hsa-miR-1300.

4. Verfahren zur Bestimmung des fernmetastasenfreien Überlebens bei Dickdarmkrebspatienten nach chirurgischer Behandlung, bestehend aus der Bestimmung der Menge an microRNAs, umfassend die Isolierung von RNA aus Dickdarmkrebsgewebe und die Quantifizierung der microRNA, **dadurch gekennzeichnet, dass**
5-mikroRNAs: hsa-miR-1296, hsa-miR-135b, hsa-miR-539, hsa-miR-572, hsa-miR-185 mittels quantitativer Reverse-Transkriptase-Polymerase-Kettenreaktion oder Hybridisierungsverfahren, wie die Nanostring-Technologie, quantifiziert werden, und ferner werden die erhaltenen microRNA-Expressionswerte in die microRNA-Expressionswerte im Modell zur Vorhersage des Rezidivrisikos eingegeben, und der Risikoscore RS für das Rezidiv von Darmkrebs wird als logit(RS)-Funktion einschließlich eines Gewichtungsfaktors für den Beitrag jeder analysierten microRNA gemäß der Formel berechnet: logit (RS) = -3. 61 - 0,72 × hsa-miR-135b + 1,45 × hsa-miR-185 - 0,59 × hsa-miR-539 - 0,61 × hsa-miR-1296 + 0,68 × hsa-miR-572.

## Revendications

1. Procédé pour déterminer la survie sans métastases à distance chez des patients atteints de cancer du colon après un traitement chirurgical consistant à déterminer la quantité des micro-ARNs comprenant l'isolation de l'ARN du tissu de cancer colorectal, la quantification de micro-ARN, **caractérisée en ce que**
10-micro-ARNs : hsa-miR-155, hsa-miR-210, hsa-miR-539, hsa-miR-1296, hsa-miR-1303, hsa-miR-151-5p, hsa-miR-497, hsa-miR-552, hsa-miR-572, hsa-miR-939 sont quantifiés par la réaction en chaîne par polymérase quantitative de transcriptase inverse ou par méthode d'hybridation, telle que une technologie de Nanochaîne, et en outre les valeurs de expression de micro-ARN obtenues sont saisies dans les valeurs de expression de micro-ARN dans le modèle de prédiction du risque de récurrence et la cote de risque RS de récurrence du cancer colorectal est calculée comme la fonction logit(RS) incluant un facteur de pondération d'apport de chacun micro-ARN analysé selon la formule suivante : logit(RS) = 0,95 + 0,86 × hsa-miR-155 + 0,73 × hsa-miR-210 - 0,84 × hsa-miR-539 - 0,47 × hsa-miR-1296 + 0,65 × hsa-miR-1303 - 0,17 × hsa-miR-151-5p - 0,27 × hsa-miR-497 - 0.35 × hsa-miR-552 + 0,55 × hsa-miR-572 - 0,07 × hsa-miR-939.

2. Procédé pour déterminer la survie sans métastases à distance chez des patients atteints de cancer du colon après un traitement chirurgical consistant à déterminer la quantité des micro-ARNs comprenant l'isolation de l'ARN du tissu de cancer colorectal, la quantification de micro-ARN, **caractérisée en ce que**
9-micro-ARNs : hsa-miR-135b, hsa-miR-185, hsa-miR-539, hsa-miR-1296, hsa-miR-572, hsa-miR-939, hsa-miR-155, hsa-miR-497, hsa-miR-1300 sont quantifiés par la réaction en chaîne par polymérase quantitative de transcriptase inverse ou par méthode d'hybridation, telle que une technologie de Nanochaîne, et en outre les valeurs de expression de micro-ARN obtenues sont saisies dans les valeurs de expression de micro-ARN dans le modèle de prédiction du risque de récurrence et la cote de risque RS de récurrence du cancer colorectal est calculée comme la fonction logit(RS) incluant un facteur de pondération d'apport de chacun micro-ARN analysé selon la formule suivante : logit (RS) = -1,52 - 0,78 × hsa-miR-135b + 1,19 × hsa-miR-185 - 0,57 × hsa-miR-539 - 0,63 × hsa-miR-1296 + 0,61 × hsa-miR-572 + 0,25 × hsa-miR-939 + 0,58 × hsa-miR-155 - 0,35 × hsa-miR-497 + 0,14 × hsa-miR-1300.

3. Procédé pour déterminer la survie sans métastases à distance chez des patients atteints de cancer du colon après un traitement chirurgical consistant à déterminer la quantité des micro-ARNs comprenant l'isolation de l'ARN du tissu de cancer colorectal, la quantification de micro-ARN, **caractérisée en ce que**
7-micro-ARNs : hsa-miR-135b, hsa-miR-185, hsa-miR-539, hsa-miR-1296, hsa-miR-572, hsa-miR-939, hsa-miR-1300 sont quantifiés par la réaction en chaîne par polymérase quantitative de transcriptase inverse ou par méthode d'hybridation, telle que une technologie de Nanochaîne, et en outre les valeurs de expression de micro-ARN obtenues sont saisies dans les valeurs de expression de micro-ARN dans le modèle de prédiction du risque de récurrence et la cote de risque RS de récurrence du cancer colorectal est calculée comme la fonction logit(RS) incluant un facteur de pondération d'apport de chacun micro-ARN analysé selon la formule suivante : logit(RS) = -2,78 - 0,81 × hsa-miR-135b + 1,45 × hsa-miR-185 - 0,63 × hsa-miR-539 - 0,71 × hsa-miR-1296 + 0,44 × hsa-miR-572 + 0,34 × hsa-miR-939 + 0,13 × hsa-miR-1300.

4. Procédé pour déterminer la survie sans métastases à distance chez des patients atteints de cancer du colon après un traitement chirurgical consistant à déterminer la quantité des micro-ARNs comprenant l'isolation de l'ARN du tissu de cancer colorectal, la quantification de micro-ARN, **caractérisée en ce que**
5-micro-ARNs : hsa-miR-1296, hsa-miR-135b, hsa-miR-539, hsa-miR-572, hsa-miR-185 sont quantifiés par la réaction en chaîne par polymérase quantitative de transcriptase inverse ou par méthode d'hybridation, telle que une technologie de Nanochaîne, et en outre les valeurs de expression de micro-ARN obtenues sont saisies dans les valeurs de expression de micro-ARN dans le modèle de prédiction du risque de récurrence et la cote de risque RS de récurrence du cancer colorectal est calculée comme la fonction logit(RS) incluant un facteur de pondération d'apport de chacun micro-ARN analysé selon la formule suivante : logit(RS) = -3,61 - 0,72 × hsa-miR-135b + 1,45 × hsa-miR-185 - 0,59 × hsa-miR-539 - 0,61 × hsa-miR-1296 + 0,68 × hsa-miR-572.
